# EUROPEAN PATENT SPECIFICATION

(11) **EP 2 094 850 B1**
(45) Date of publication and mention of the grant of the patent: **12.06.2013**
(21) Application number: 07852282.8
(22) Date of filing: 03.12.2007
(51) Int. Cl.: G01N 33/574, C12N 9/12

(54) **CANCER-RELATED PROTEIN KINASES**
PROTEINKINASEN IN VERBINDUNG MIT KREBS
PROTÉINE KINASES LIÉES AU CANCER

(30) Priority: 01.12.2006 US 868173 P
(43) Date of publication of application: 02.09.2009
(73) Proprietor: Agency for Science, Technology And Research, Singapore 138632 (SG)
(72) Inventor: ULLRICH, Axel, 80331 Munich (DE); RUHE, Jens, Singapore 109258 (SG); HART, Stefan, Singapore 597156 (SG); STREIT, Sylvia, Singapore 109258 (SG); WONG, Chee Hong, Singapore 141052 (SG); CHUA, Boon Tin, Singapore 610151 (SG); HO, Han Kiat, Singapore 689105 (SG)
(74) Representative: Viering, Jentschura & Partner
(86) International application number: PCT/SG2007/000412
(87) International publication number: WO 2008/066498

(56) References cited:
- WO-A1-2004/074485
- WO-A2-02/31198
- WO-A2-03/065006
- WO-A2-2005/031001
- JP-A- 2005 198 640
- US-A1- 2006 194 265
- STREIT SYLVIA ET AL: "Involvement of the FGFr4 Arg388 allele in head and neck squamous cell carcinoma" INTERNATIONAL JOURNAL OF CANCER, JOHN WILEY & SONS, INC, UNITED STATES, SWITZERLAND, GERMANY LNKD- DOI:10.1002/IJC.20204, vol. 111, no. 2, 20 August 2004 (2004-08-20), pages 213-217, XP002517570 ISSN: 0020-7136 [retrieved on 2004-04-12]
- STREIT S ET AL: "FGFR4 Arg388 allele correlates with tumour thickness and FGFR4 protein expression with survival of melanoma patients" BRITISH JOURNAL OF CANCER 20060619 GB LNKD- DOI:10.1038/SJ.BJC.6603181, vol. 94, no. 12, 19 June 2006 (2006-06-19) , pages 1879-1886, XP002577219
- THUSSBAS CHRISTOPH ET AL: "FGFR4 ARG388 ALLELE IS ASSOCIATED WITH RESISTANCE TO ADJUVANT THERAPY IN PRIMARY BREAST CANCER" JOURNAL OF CLINICAL ONCOLOGY, AMERICAN SOCIETY OF CLINICAL ONCOLOGY, US LNKD- DOI:10.1200/JCO.2005.04.8587, vol. 24, no. 23, 10 August 2006 (2006-08-10), pages 3747-3755, XP008076988 ISSN: 0732-183X
- EZZAT S. ET AL.: 'Targeted expression of a human pituitary tumor-derived isoform of FGF receptor-4 recapitulates pituitary tumorigenesis' JOURNAL OF CLINICAL INVESTIGATION vol. 109, no. 1, 2002, pages 69 - 78, XP008109194
- RON D. ET AL.: 'Fibroblast Growth Factor Receptor 4 Is a High Affinity Receptor for Both Acidic and Basic Fibroblast Growth Factor but Not for Keratinocyte Growth Factor' JOURNAL OF BIOLOGICAL CHEMISTRY vol. 268, no. 8, 1993, pages 5388 - 5394, XP008109195
- KOSTRZEWA M. AND MOLLER U.: 'Genomic structure and complete sequence of the human FGFR4 gene' MAMMALIAN GENOME vol. 9, no. 2, 1998, pages 131 - 135, XP002441350

### SEQUENCE LISTING

<110> Agency f or Sci ence, Technology and Research (ASTAR)
<120> Cancer - Rel at ed Pr ot ei n Kinases
<130> P101290
<150> US 60/ 868, 173
   <151> 2006- 12- 01
<160> 2
<170> Patent In version 3.3
<210> 133
   <211> 802
   <212> PRT
   <213> Homo sapiens
<400> 133
   Met Arg Leu Leu Leu Ala Leu Leu Gly Val Leu Leu Ser Val Pro Gly
   1 5 10 15
   Pro Pro Val Leu Ser Leu Glu Ala Ser Glu Glu Val Glu Leu Glu Pro
   20 25 30
   Cys Leu Ala Pro Ser Leu Glu Gln Gln Glu Gln Glu Leu Thr Val Ala
   35 40 45
   Leu Gly Gln Pro Val Arg Leu Cys Cys Gly Arg Ala Glu Arg Gly Gly
   50 55 60
   His Trp Tyr Lys Glu Gly Ser Arg Leu Ala Pro Ala Gly Arg Val Arg
   65 70 75 80
   Gly Trp Arg Gly Arg Leu Glu Ile Ala Ser Phe Leu Pro Glu Asp Ala
   85 90 95
   Gly Arg Tyr Leu Cys Leu Ala Arg Gly Ser Met Ile Val Leu Gln Asn
   100 105 110
   Leu Thr Leu Ile Thr Gly Asp Ser Leu Thr Ser Ser Asn Asp Asp Glu
   115 120 125
   Asp Pro Lys Ser His Arg Asp Pro Ser Asn Arg His Ser Tyr Pro Gln
   130 135 140
   Gln Ala Pro Tyr Trp Thr His Pro Gln Arg Met Glu Lys Lys Leu His
   145 150 155 160
   Ala Val Pro Ala Gly Asn Thr Val Lys Phe Arg Cys Pro Ala Ala Gly
   165 170 175
   Asn Pro Thr Pro Thr Ile Arg Trp Leu Lys Asp Gly Gln Ala Phe His
   180 185 190
   Gly Glu Asn Arg Ile Gly Gly Ile Arg Leu Arg His Gln His Trp Ser
   195 200 205
   Leu Val Met Glu Ser Val Val Pro Ser Asp Arg Gly Thr Tyr Thr Cys
   210 215 220
   Leu Val Glu Asn Ala Val Gly Ser Ile Arg Tyr Asn Tyr Leu Leu Asp
   225 230 235 240
   Val Leu Glu Arg Ser Pro His Arg Pro Ile Leu Gln Ala Gly Leu Pro
   245 250 255
   Ala Asn Thr Thr Ala Val Val Gly Ser Asp Val Glu Leu Leu Cys Lys
   260 265 270
   Val Tyr Ser Asp Ala Gln Pro His Ile Gln Trp Leu Lys His Ile Val
   275 280 285
   Ile Asn Gly Ser Ser Phe Gly Ala Asp Gly Phe Pro Tyr Val Gln Val
   290 295 300
   Leu Lys Thr Ala Asp Ile Asn Ser Ser Glu Val Glu Val Leu Tyr Leu
   305 310 315 320
   Arg Asn Val Ser Ala Glu Asp Ala Gly Glu Tyr Thr Cys Leu Ala Gly
   325 330 335
   Asn Ser Ile Gly Leu Ser Tyr Gln Ser Ala Trp Leu Thr Val Leu Pro
   340 345 350
   Glu Glu Asp Pro Thr Trp Thr Ala Ala Ala Pro Glu Ala Arg Cys Thr
   355 360 365
   Asp Ile Ile Leu Tyr Ala Ser Gly Ser Leu Ala Leu Ala Val Leu Leu
   370 375 380
   Leu Leu Ala Gly Leu Tyr Arg Gly Gln Ala Leu His Gly Arg His Pro
   385 390 395 400
   Arg Pro Pro Ala Thr Val Gln Lys Leu Ser Arg Phe Pro Leu Ala Arg
   405 410 415
   Gln Phe Ser Leu Glu Ser Gly Ser Ser Gly Lys Ser Ser Ser Ser Leu
   420 425 430
   Val Arg Gly Val Arg Leu Ser Ser Ser Gly Pro Ala Leu Leu Ala Gly
   435 440 445
   Leu Val Ser Leu Asp Leu Pro Leu Asp Pro Leu Trp Glu Phe Pro Arg
   450 455 460
   Asp Arg Leu Val Leu Gly Lys Pro Leu Gly Glu Gly Cys Phe Gly Gln
   465 470 475 480
   Val Val Arg Ala Glu Ala Phe Gly Met Asp Pro Ala Arg Pro Asp Gln
   485 490 495
   Ala Ser Thr Val Ala Val Lys Met Leu Lys Asp Asn Ala Ser Asp Lys
   500 505 510
   Asp Leu Ala Asp Leu Val Ser Glu Met Glu Val Met Lys Leu Ile Gly
   515 520 525
   Arg His Lys Asn Ile Ile Asn Leu Leu Gly Val Cys Thr Gln Glu Gly
   530 535 540
   Pro Leu Tyr Val Ile Val Glu Cys Ala Ala Lys Gly Asn Leu Arg Glu
   545 550 555 560
   Phe Leu Arg Ala Arg Arg Pro Pro Gly Pro Asp Leu Ser Pro Asp Gly
   565 570 575
   Pro Arg Ser Ser Glu Gly Pro Leu Ser Phe Pro Val Leu Val Ser Cys
   580 585 590
   Ala Tyr Gln Val Ala Arg Gly Met Gln Tyr Leu Glu Ser Arg Lys Cys
   595 600 605
   Ile His Arg Asp Leu Ala Ala Arg Asn Val Leu Val Thr Glu Asp Asn
   610 615 620
   Val Met Lys Ile Ala Asp Phe Gly Leu Ala Arg Gly Val His His Ile
   625 630 635 640
   Asp Tyr Tyr Lys Lys Thr Ser Asn Gly Arg Leu Pro Val Lys Trp Met
   645 650 655
   Ala Pro Glu Ala Leu Phe Asp Arg Val Tyr Thr His Gln Ser Asp Val
   660 665 670
   Trp Ser Phe Gly Ile Leu Leu Trp Glu Ile Phe Thr Leu Gly Gly Ser
   675 680 685
   Pro Tyr Pro Gly Ile Pro Val Glu Glu Leu Phe Ser Leu Leu Arg Glu
   690 695 700
   Gly His Arg Met Asp Arg Pro Pro His Cys Pro Pro Glu Leu Tyr Gly
   705 710 715 720
   Leu Met Arg Glu Cys Trp His Ala Ala Pro Ser Gln Arg Pro Thr Phe
   725 730 735
   Lys Gln Leu Val Glu Ala Leu Asp Lys Val Leu Leu Ala Val Ser Glu
   740 745 750
   Glu Tyr Leu Asp Leu Arg Leu Thr Phe Gly Pro Tyr Ser Pro Ser Gly
   755 760 765
   Gly Asp Ala Ser Ser Thr Cys Ser Ser Ser Asp Ser Val Phe Ser His
   770 775 780
   Asp Pro Leu Pro Leu Gly Ser Ser Ser Phe Pro Phe Gly Ser Gly Val
   785 790 795 800
   Gln Thr
<210> 402
   <211> 2409
   <212> DNA
   <213> Homo sapiens
<400> 402
   atgcggctgc tgctggccct gttgggggtc ctgctgagtg tgcctgggcc tccagtcttg 60
   tccctggagg cctctgagga agtggagctt gagccctgcc tggctcccag cctggagcag 120
   caagagcagg agctgacagt agcccttggg cagcctgtgc gtctgtgctg tgggcgggct 180
   gagcgtggtg gccactggta caaggagggc agtcgcctgg cacctgctgg ccgtgtacgg 240
   ggctggaggg gccgcctaga gattgccagc ttcctacctg aggatgctgg ccgctacctc 300
   tgcctggcac gaggctccat gatcgtcctg cagaatctca ccttgattac aggtgactcc 360
   ttgacctcca gcaacgatga tgaggacccc aagtcccata gggacctctc gaataggcac 420
   agttaccccc agcaagcacc ctactggaca cacccccagc gcatggagaa gaaactgcat 480
   gcagtacctg cggggaacac cgtcaagttc cgctgtccag ctgcaggcaa ccccacgccc 540
   accatccgct ggcttaagga tggacaggcc tttcatgggg agaaccgcat tggaggcatt 600
   cggctgcgcc atcagcactg gagtctcgtg atggagagcg tggtgccctc ggaccgcggc 660
   acatacacct gcctggtaga gaacgctgtg ggcagcatcc gctataacta cctgctagat 720
   gtgctggagc ggtccccgca ccggcccatc ctgcaggccg ggctcccggc caacaccaca 780
   gccgtggtgg gcagcgacgt ggagctgctg tgcaaggtgt acagcgatgc ccagccccac 840
   atccagtggc tgaagcacat cgtcatcaac ggcagcagct tcggagccga cggtttcccc 900
   tatgtgcaag tcctaaagac tgcagacatc aatagctcag aggtggaggt cctgtacctg 960
   cggaacgtgt cagccgagga cgcaggcgag tacacctgcc tcgcaggcaa ttccatcggc 1020
   ctctcctacc agtctgcctg gctcacggtg ctgccagagg aggaccccac atggaccgca 1080
   gcagcgcccg aggccaggtg tacggacatc atcctgtacg cgtcgggctc cctggccttg 1140
   gctgtgctcc tgctgctggc cgggctgtat cgagggcagg cgctccacgg ccggcacccc 1200
   cgcccgcccg ccactgtgca gaagctctcc cgcttccctc tggcccgaca gttctccctg 1260
   gagtcaggct cttccggcaa gtcaagctca tccctggtac gaggcgtgcg tctctcctcc 1320
   agcggccccg ccttgctcgc cggcctcgtg agtctagatc tacctctcga cccactatgg 1380
   gagttccccc gggacaggct ggtgcttggg aagcccctag gcgagggctg ctttggccag 1440
   gtagtacgtg cagaggcctt tggcatggac cctgcccggc ctgaccaagc cagcactgtg 1500
   gccgtcaaga tgctcaaaga caacgcctct gacaaggacc tggccgacct ggtctcggag 1560
   atggaggtga tgaagctgat cggccgacac aagaacatca tcaacctgct tggtgtctgc 1620
   acccaggaag ggcccctgta cgtgatcgtg gagtgcgccg ccaagggaaa cctgcgggag 1680
   ttcctgcggg cccggcgccc cccaggcccc gacctcagcc ccgacggtcc tcggagcagt 1740
   gaggggccgc tctccttccc agtcctggtc tcctgcgcct accaggtggc ccgaggcatg 1800
   cagtatctgg agtcccggaa gtgtatccac cgggacctgg ctgcccgcaa tgtgctggtg 1860
   actgaggaca atgtgatgaa gattgctgac tttgggctgg cccgcggcgt ccaccacatt 1920
   gactactata agaaaaccag caacggccgc ctgcctgtga agtggatggc gcccgaggcc 1980
   ttgtttgacc gggtgtacac acaccagagt gacgtgtggt cttttgggat cctgctatgg 2040
   gagatcttca ccctcggggg ctccccgtat cctggcatcc cggtggagga gctgttctcg 2100
   ctgctgcggg agggacatcg gatggaccga cccccacact gccccccaga gctgtacggg 2160
   ctgatgcgtg agtgctggca cgcagcgccc tcccagaggc ctaccttcaa gcagctggtg 2220
   gaggcgctgg acaaggtcct gctggccgtc tctgaggagt acctcgacct ccgcctgacc 2280
   ttcggaccct attccccctc tggtggggac gccagcagca cctgctcctc cagcgattct 2340
   gtcttcagcc acgaccccct gccattggga tccagctcct tccccttcgg gtctggggtg 2400
   cagacatga                                                         2409

## Description

### FIELD OF THE INVENTION

The present invention relates to mutant protein kinases, nucleotide sequences encoding the mutated protein kinases, their use for the diagnosis of various kinase-related diseases and conditions and the design and identification of novel protein kinase inhibitors.

### BACKGROUND OF THE INVENTION

The following description of the background of the invention is provided to aid in understanding the invention, but is not admitted to be or to describe prior art to the invention.

Cancer is the second most common cause of death in developed countries and is a rising health problem in less developed parts of the world. The diagnosis of cancer is connected to great physical and mental suffering for affected individuals and poses a significant burden on the health care system. For many tumors, conventional management strategies, such as surgery, radiation therapy and chemotherapy, have high toxicity with limited efficacy. Thus, an in-depth understanding of the molecular genetics underlying individual malignancies will greatly facilitate the cancer therapeutic problem is now commonly accepted among investigators in the field.

Autonomous cell growth resulting in tissue invasion and metastasis is the defining feature of all malignant neoplasms. Cancers do not necessarily arise solely as a result of an accelerated rate of cell proliferation. Rather they are the consequence of an imbalance between the rate of cell-cycle progression (cell division) and cell growth (cell mass) on one hand and programmed cell death (apoptosis) on the other. Researchers now recognize that aberrant cellular signal transduction pathways play a vital role in driving this imbalance and hence in malignant transformation.

Cellular signal transduction is a fundamental mechanism whereby external stimuli that regulate diverse cellular processes are relayed to the interior of cells. One of the key biochemical mechanisms of signal transduction involves the reversible phosphorylation of proteins, which enables regulation of the activity of mature proteins by altering their structure and function.

Thus, one of the most critical groups of signaling molecules involved in normal and abnormal cellular regulation are the protein kinases, a family of enzymes that catalyze the phosphorylation of amino acid residues of various target molecules. This process controls fundamental cellular processes including cell cycle, migration, metabolism, proliferation, differentiation, and survival.

Protein kinases are one of the largest families of eukaryotic proteins with several hundred known members. These proteins share a 250-300 amino acid long kinase domain that can be subdivided into 12 distinct subdomains that includes the common catalytic core structure. These conserved protein motifs have recently been exploited using PCR-based cloning strategies leading to a significant expansion of the known kinases.

The best-characterized protein kinases in eukaryotes phosphorylate proteins on the hydroxyl moiety of serine, threonine and/or tyrosine residues. These kinases largely fall into two groups, those specific for phosphorylating serines and threonines, and those specific for phosphorylating tyrosines. Some kinases, referred to as "dual specificity" kinases, are able to phosphorylate on tyrosine as well as serine/threonine residues.

Protein kinases can also be characterized by their location within the cell. Some kinases are transmembrane receptor-type proteins capable of directly altering their catalytic activity in response to the external environment such as the binding of a ligand. Others are non-receptor-type proteins lacking any transmembrane domain. They can be found in a variety of cellular compartments from the inner surface of the cell membrane to the nucleus.

Many kinases are involved in regulatory cascades wherein their substrates may include other kinases whose activities are regulated by their phosphorylation state. Ultimately the activity of some downstream effector is modulated by phosphorylation resulting from activation of such a pathway.

Protein tyrosine phosphorylation, mediated by protein tyrosine kinases, is a key mechanism underlying signal transduction pathways that regulate fundamental cellular processes such as proliferation, differentiation, motility and cell survival. Deregulation of kinase activity, caused by genetic alterations, modulated expression levels, or the loss of negative regulatory control mechanisms has been described for various members of the tyrosine kinase family, and in many cases has been implicated in the development of human cancer (Blume-Jensen,P., & Hunter, T., Nature 411, 355-365 (2001)). Consequently, tyrosine kinases have become rational targets for therapeutic intervention using both monoclonal antibodies and small molecule drugs.

First hints for genetically modified tyrosine kinases to be involved in the development of cancer came from viral oncogenes which in several cases have been shown to represent altered versions of cellular receptor tyrosine kinases. The avian erythroblastosis gene v-erbB for example has been identified as a truncated and mutated version of the human epidermal growth factor receptor EGFR (e.g. Downward, J., et al. Nature 311, 483-485, (1984)), which for the first time connected an animal oncogene with a human gene that encoded a cell growth controlling membrane protein, Furthermore, v-fms was found to represent a deleted form of the macrophage CSF-1 receptor (Coussens, L., et al. Nature 320, 277-280 (1986); Sherr, C. J., et al., Cell 41, 665-676 (1985)), and the identified truncations, deletions and mutations were speculated to form the genetic basis for the conversion of a proto-oncogene into an oncogene that can cause malignant cancer in animals.

These observations stimulated a massive search for genetic alterations of tyrosine kinases in human cancer. Several deletions and point mutations were described that result in increased catalytic activity of the EGFR. The most prevalent in tumors was found to be EGFRvIII, an EGFR deletion mutant that lacks exons 2-7, which can arise from gene rearrangement or alternative mRNA splicing (Malden, L. T., et al., Cancer Res 48, 2711-2714 (1988)). Amplification of the HER2 gene (Coussens, L., et al., Science 230, 1132-1139 (1985)) - another member of the EGFR family - was discovered as a genetic abnormality occurring in 30% of invasive human breast cancer, and a significant correlation between HER2 overexpression in tumors and reduced patient survival could be demonstrated (Slamon, D. J., et al., Science 235,177-182 (1987)). These findings established HER2 as a prognostic marker and led to evaluate the concept of target-specific cancer therapy. In 1998, it culminated in the FDA-approval of Herceptin, a humanized monoclonal against HER2 and the first targeted anti-kinase therapeutic agent based on genomic research.

Since then it became more and more obvious that even single genetic changes were able to mediate oncogenic potential to a given kinase. This was first shown for neu, the rat homologue of the human HER2 gene, where the replacement of a single valine within the transmembrane domain by glutamic acid resulted in activation of p185 and tumorigenic activity of the modified neu proto-oncogene (Bargmann, C. I., et al., Cell 45, 649-657 (1986)). Other very well known genetic variations in tyrosine kinase genes that are associated with cancer are the BCR-ABL oncogene, a reciprocal translocation between chromosomes 9 and 22 in chronic myelogenous leukaemia (CML), and KIT receptor point mutations in gastrointestinal stromal tumors (GISTs) (Corless, C. L., et al., J Mol Diagn. 6, 366-370 (2004)) - both being targeted by the small molecule imatinib (Gleevec, Novartis) (Demetri, G. D., Eur. J Cancer 38 Suppl 5, S52-S59 (2002); Peggs, K. & Mackinnon, S., N. Engl. J Med. 348, 1048-1050 (2003)).

In addition to their significant role in disease initiation or progression, specific mutations could be shown to mediate and thus predict sensitivity towards specific small molecule inhibitors such as imatinib or gefitinib (Iressa, AstraZeneca). Two independent groups recently described the identification of mutations clustered around the ATP binding pocket of the EGFR kinase domain and demonstrated their occurrence primarily in patients with Iressa-responsive lung cancer (Lynch, T. J., et al., N. Engl. J Med. 350, 2129-2139 (2004); Paez, J. G., et al., Science 304, 1497-1500 (2004)).

Therefore, significant efforts have been undertaken to screen for mutations in tyrosine kinase encoding genes on the level, of genomic DNA, and comprehensive studies focusing on the kinase domain in colorectal cancer (Bardelli, A., et al., Science 300, 949 (2003)) or selected tumor types (Bignell, G., et al., Genes, Chromosomes & Cancer 45:42-46 (2006) (2006); Davies, H., et al., et al., Cancer Res. 65:17, 7591-7595 (2005); Stephens, P., et al., Nature 431:525-526 (2004)) have recently been reported.

Streit Sylvia et al., International Journal of Cancer, John Wiley & Sons, Inc, US, CH, GR, Vol. 111, No. 2, 20 August 2004, pages 213-217 describe the investigation of the expression pattern of FGFR4 and the clinical significance of identified Gly/Arg polymorphism (388) in head and neck squamous cell carcinomase (HNSCCs) of the oral cavity and the oropharynx.

Streit et al., British Journal of Cancer, 2006, Vol. 94, pages 1879-1886 describe the analysis of the protein expression of FGF receptor subtype 4 in 137 melanoma tissues of different progression stages by immunohistochemistry and found in conclusion that the Arg388 genotype and the protein expression of FGFR4 may be potential markers for progression of melanoma.

Thussbas et al., Journal of Clinical Oncology, 2006, Vol. 24, No. 23, pages 3747-3755 describe the immunohistochemical assessment of FGFR4 and HER2 expression in tissue microarrays by performing polymerase chain reaction restriction fragment length polymorphism analysis of germ-line polymorphism in uninvolved lymph nodes.

For decades the traditional approach to identify cancer genes was to hand-pick likely candidates and then search for mutations within them. But since the completion of the draft human genome sequence in 2000, scientists have had the tools to go after cancer mutations in a more global and systematic way.

Scientists of the post-genome era can examine the sequences of thousands of genes in cancer cells. But because of the expense and technical limitations of current sequencing technologies, groups have for now been focusing on specific sets of genes rather than attempting to go after all of them at once.

DNA sequencing, although a major component, is only one part of the process of finding cancer mutations. Indeed cancer can arise from small mutations in the DNA sequences, changes in the number of copies of specific genes, rearrangements affecting entire chromosomes, and even from tumor viruses that land inside or next to human genes. In addition, epigenetic changes are also thought to play a role in cancer.

However, the large variation in molecular changes from one tumor to another, and even within the same tumor from one cell to another, has long been an obstacle for the development of effective therapies. Recent studies provided the first clear demonstration of the vast array of mutations present in cancer cells and identified close to 200 mutations in protein kinase genes in lung tumors, indicating that many mutated protein kinases may be contributing to lung cancer development but that mutations in any one gene are infrequent.

For some tumors one will thus find high frequency mutations that will make good drug targets, but many will be a mixture of different low frequency mutations.

Beyond the challenge of obtaining accurate DNA sequence on such large scale, researches have to sift through the hundreds of changes identified in any one set of genes and determine which are specific to cancer and which are normal changes that occur in DNA of any individual (polymorphisms).

This problem can be addressed by comparing each cancer sequence to that of DNA taken from normal cells of the same patient. Polymorphisms should be present in both samples whereas cancer-specific changes should be present only in the cancer DNA.

The present invention relates to the identification of protein kinase mutations prevalent in human malignancies as well as methods of use of such mutated protein kinases. The invention further relates to germline variations in kinase genes that are related to tumor development and progression.

### SUMMARY OF THE INVENTION

In its broadest sense teh present application is as defined in the appended claims. Thus, in a first aspect the invention provides an isolated, enriched, or purified nucleic acid molecule. The nucleic acid molecule encodes a mutant of a protein kinase polypeptide. The protein kinase is FGFR4. Further, the mutant of the protein kinase polypeptide encoded by the nucleic acid molecule includes the mutation of FGFR4 Y367C (SEQ ID No. 133).

In a second aspect invention provides a method of identifying a cell that has a predisposition to transform into a cancer cell. The method includes either identifying the amino acid at position 367 of the expressed protein kinase FGFR4. Where the method includes identifying the amino acid at position 367 of the expressed protein kinase FGFR4, the presence of Cysteine at position 367 of the expressed protein kinase FGFR4 instead of Tyrosine indicates an increased predisposition to transform into a cancer cell.

The nucleic acid may be isolated from a natural source by cDNA cloning or by subtractive hybridization. The natural source may be mammalian, for example, of murine, human, porcine, canine, or bovine origin. The polypeptide can be isolated from every suitable sample, including cultured cells, a biopsy, blood, semen, or any tissue derived from an organ, for example, skin, liver, pancreas to name only a few illustrative examples. In another aspect, the nucleic acid may be synthesized by the triester method or by using an automated DNA synthesizer.

In other embodiments, the nucleic acid molecule may be of recombinant origin or the nucleic acid molecule may be RNA or DNA.

In a third aspect, the invention provides a nucleic acid probe for the detection of a nucleic acid molecule that encodes a mutant kinase polypeptide in a sample. The mutant kinase polypeptide is FGFR4. The mutant kinase polypeptide encoded by said nucleic acid molecule comprises the mutation FGFR4 Y367C (SEQ ID No: 402). The nucleic acid probe is configured to bind to the FGFR4 having the Y367C mutation and not to a wild type FGF4 or any other FGF4 which does not have the Y367C mutation. Thus, the nucleic acid probe may include, consist essentially of or consist of a nucleotide base sequence that will hybridize to the mutated region of the nucleic acid sequence set forth in any of SEQ ID No: 402

Methods for using the probes of the invention include detecting the presence or amount of a mutant of FGFR4 in a sample by contacting the sample with a nucleic acid probe under conditions such that hybridization occurs and detecting the presence or amount of the probe bound to nucleic acid molecules encoding the mutant of FGFR4. The mutant of FGF4 encoded by the nucleic acid molecule comprises the mutation FGFR4 Y367C (SEQ ID No. 133). The nucleic acid duplex formed between the probe and a nucleic acid sequence coding for a nucleic acid molecules encoding the mutant of FGFR4may be used in the identification of the sequence of the nucleic acid detected. In certain embodiment, kits for performing such methods may be constructed to include a container means having disposed therein a nucleic acid probe.

The present invention also relates to the use of the mutant kinase polypeptide, the nucleic acids encoding the mutant kinase polypeptide, and the nucleic acid probes of the invention as molecular markers for the diagnosis of proliferative diseases or disorders in a subject. Such a method may also be useful to predict the risk of cancer with high predictive accuracy and/or to choose an adequate therapy. Moreover, such a method may also be useful to monitor the course of a treatment regimen and/or to predict the risk or cancer recurrence.

Thus, the present invention also encompasses a method that allows predicting or diagnosing proliferative diseases or disorders, such as cancer, in a subject comprising the steps of (a) obtaining a biological sample from the subject; and (b) detecting the expression of one or more nucleic acid molecules encoding the mutant kinase polypeptide of the invention in said sample.

In still another aspect, the invention provides a recombinant cell or tissue comprising a nucleic acid molecule encoding a kinase polypeptide of, including the mutation FGFR4 Y367C. In such cells, the nucleic acid may be under the control of the genomic regulatory elements, or may be under the control of one or more heterologous regulatory elements including a heterologous promoter. In certain embodiments, the kinase polypeptide may be a fragment of the protein encoded by the amino acid sequence set forth in SEQ ID No: 133, or the corresponding full-length amino acid sequence, wherein said fragment includes the mutated region.

In still another aspect, the invention provides an isolated, enriched, or purified mutant kinase polypeptide FGFR4, including the mutation FGFR4 Y367C (SEQ ID No. 133).

In some embodiments, the mutant kinase polypeptide may be a fragment of the protein with the amino acid sequence set forth in SEQ ID No: 133, or the corresponding full-length amino acid sequences, with the proviso that the mutation is included in said fragment. Also included are variants and isoforms of the mutant kinase of the invention.

The polypeptide can be isolated from a natural source by methods well-known in the art. The natural source may be mammalian, for example, of murine, human, porcine, canine, or bovine origin. The polypeptide can be isolated from every suitable sample, including cultured cells, a biopsy, blood, semen, or any tissue derived from an organ, for example, skin, liver, pancreas to name only a few illustrative examples. In another embodiment the polypeptide may be synthesized using an automated polypeptide synthesizer.

In certain embodiments the invention includes the above mutant kinase, wherein the mutant kinase may be of recombinant origin. For example, the mutant kinase of the invention may be expressed in a heterologous expression system.

Antibodies or antibody fragments having specific binding affinity only for a mutant kinase polypeptide of the invention may be used in methods for detecting the presence and/or amount of mutant kinase polypeptide in a sample by probing the sample with the antibody under conditions suitable for kinase-antibody immunocomplex formation and detecting the presence and/or amount of the antibody conjugated to the kinase polypeptide. The antibodies of the invention are thus capable of differentiating between the mutant/variant and the native form of a kinase polypeptide. The mutant kinase polypeptide is the mutant of FGFR4 comprising the mutation FGFR4 Y367C (SEQ ID No. 133). The antibody or antibody fragment is a monoclonal or polyconal antibody or antibody fragment.

Diagnostic kits for performing such methods may be constructed to include antibodies or antibody fragments specific for the kinase as well as a conjugate of a binding partner of the antibodies or the antibodies themselves. Diagnostic kits for performing such methods may be constructed to include a first container containing the antibody and a second container having a conjugate of a binding partner of the antibody and a label, such as, for example, a radioisotope. The diagnostic kit may also include notification of an FDA approved use and instructions therefor.

An antibody or antibody fragment with specific binding affinity only for a mutant kinase polypeptide of the invention can be isolated, enriched, or purified from a prokaryotic or eukaryotic organism. Routine methods known to those skilled in the art enable production of antibodies or antibody fragments, in both prokaryotic and eukaryotic organisms. Purification, enrichment, and isolation of antibodies, which are polypeptide molecules, are described above.

In a further aspect, the invention relates to methods for identifying a compound that modulates kinase activity in vitro comprising the steps of: (a) contacting a kinase polypeptide, with a test substance; (b) measuring the activity of said polypeptide; and (c) determining whether said substance modulates the activity of said polypeptide. The kinase polypeptide is an isolated, enriched, or purified mutant kinase polypeptide, wherein said mutant kinase polypeptide is FGFR4 and wherein said mutant FGFR4 comprises the mutation FGFR4 Y367C (SEQ ID No: 133), or a fragment thereof.

In yet another aspect, the invention provides methods for diagnosis of a proliferative disease or disorder or the risk prediction of developing a proliferative disease or disorder in a subject. The disease or disorder is being characterized by an abnormality in a signal transduction pathway due to aberrant protein kinase function. The method comprises: (a) contacting a biological sample obtained from the subject with a nucleic acid probe which hybridizes under hybridization assay conditions to a target region of a nucleic acid molecule encoding a mutant kinase polypeptide; and (b) detecting the presence or amount of the probe:target region hybrid as an indication of or predisposition to the disease or disorder. The mutant kinase polypeptide is as defined above.

In certain embodiments of the invention, the disease or disorder is a proliferative disease or disorder, for example, cancer.

Hybridization conditions should be such that hybridization occurs only with the kinase genes in the presence of other nucleic acid molecules. Under stringent hybridization conditions only highly complementary nucleic acid sequences hybridize. Typically, such conditions prevent hybridization of nucleic acids having one or more mismatches in 20 contiguous nucleotides.

The diseases that could be diagnosed by detection of mutated kinase nucleic acid in a sample may include cancers. The test samples suitable for nucleic acid probing methods of the present invention include, for example, cells or nucleic acid extracts of cells, or biological fluids. The samples used in the above-described methods will vary based on the assay format, the detection method and the nature of the tissues, cells or extracts to be assayed. Methods for preparing nucleic acid extracts of cells are well-known in the art and can be readily adapted in order to obtain a sample that is compatible with the method utilized.

The summary of the invention described above is not limiting and other features and advantages of the invention will be apparent from the following detailed description of the invention, and from the claims.

### BRIEF DESCRIPTION OF THE DRAWINGS

The invention will be better understood with reference to the detailed description when considered in conjunction with the non-limiting examples and the accompanying drawings, in which:

**Fig. 1** illustrates the genetic alterations of the FGFR4 gene detected in tumor cell lines and control samples.

**Fig. 2** shows for somatic mutation, tissue distribution (BE: breast and PR: prostate) was determined. Paired numerals indicate the number of mutated and expression-positive cell lines within a given tumor type.

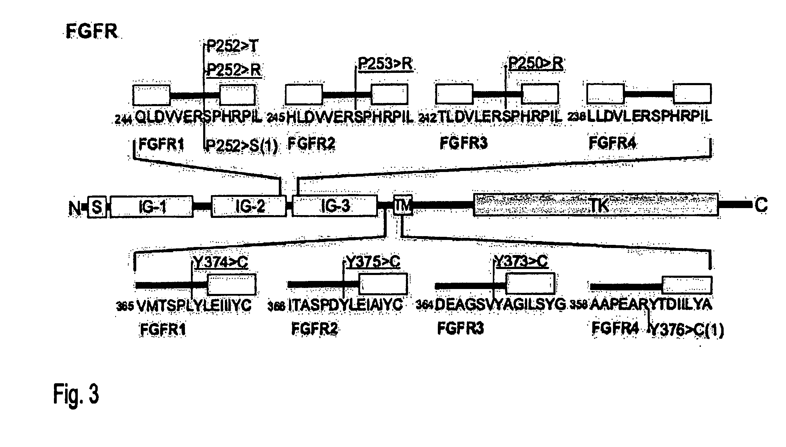

**Fig. 3** is an illustration of known and novel genetic alterations in selected genes. sequence comparison of FGFR1-4. For FGFR1-4, the general domain organization (middle) and sequence comparisons of the linker region connecting the IG-D2- and IG-D3-domain (top) as well as a part of the extracellular juxtamembrane region (bottom) are illustrated. Genetic alterations identified in the cell line screen are illustrated below, known sequence variants are depicted above the graphical representation of the domain structure. Polymorphisms are underlined and somatic mutations are not highlighted. Numbers in parenthesis indicate the number of affected non-related cell lines. (SH2: Src Homolgy 2 Domain; TK: Tyrosine Kinase Domain; S: Signal Peptide; TM: Transmembrane Domain; IG: Immunoglobulin-Like Domain).

**Fig. 4** shows overexpression of FGFR4 in hepatocellular carcinoma (HCC) patients (n= 57) in the tumor vs. the adjacent normal tissue as determined by real-time PCR.

**Fig. 5** shows the corresponding Ct values of the detected FGFR4 overexpression shown in Fig. 4.

**Fig. 6** shows elevated AFP production caused by stimulation of FGFR4 in the HCC cell line HuH7 using 50 and 100 ng/ml of the specific ligand FGF19.

**Fig. 7** shows elevated AFP production caused by stimulation of FGFR4 in the HCC cell line HepG2 using 50 and 100 ng/ml of the specific ligand FGF 19.

**Fig. 8** depicts gene silencing of FGFR4 by siRNA. A= control siRNA, B= FGFR4 siRNA.

**Fig. 9** shows AFP production in HuH7 cells aftery gene silencing of FGFR4 by siRNA.

**Fig. 10** shows AFP production in HuH7 cells after exposure to 0, 1, 5 and 10 µM of the commercially available non-selective FGFR inhibitor PD173074. SF= serum free

**Fig. 11** depicts the viability of HuH7 exposed to the FGFR inhibitor PD173074. An exquisite anti-proliferative effect can be observed.

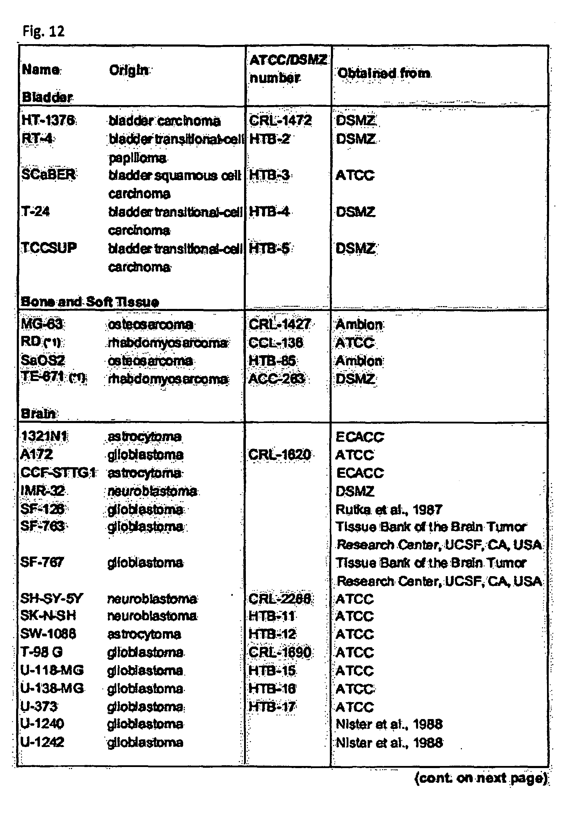

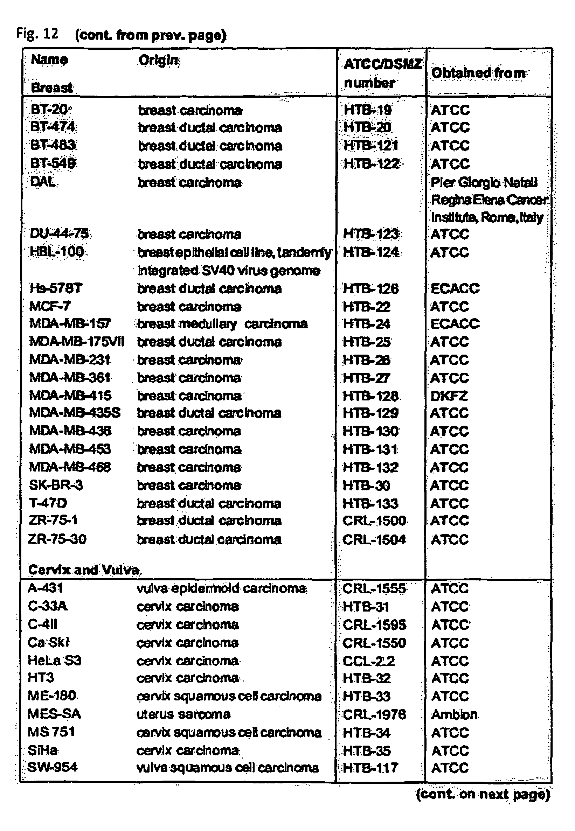

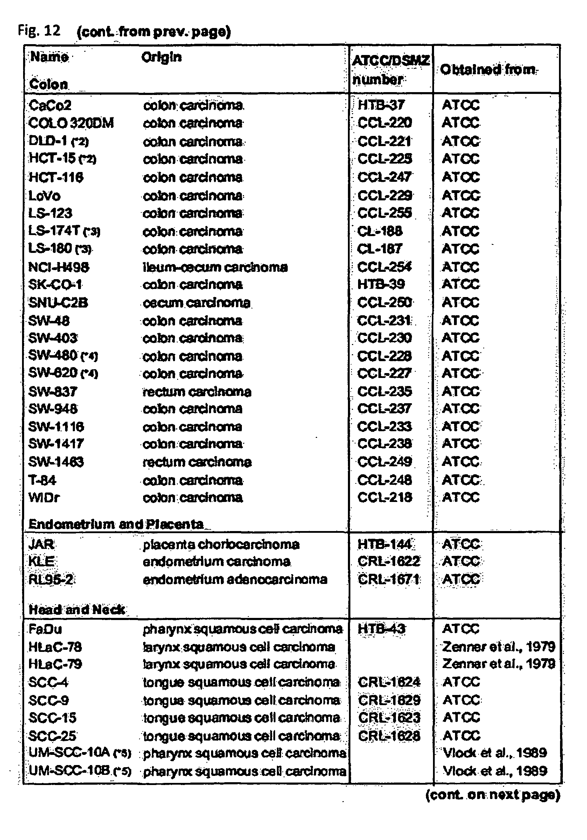

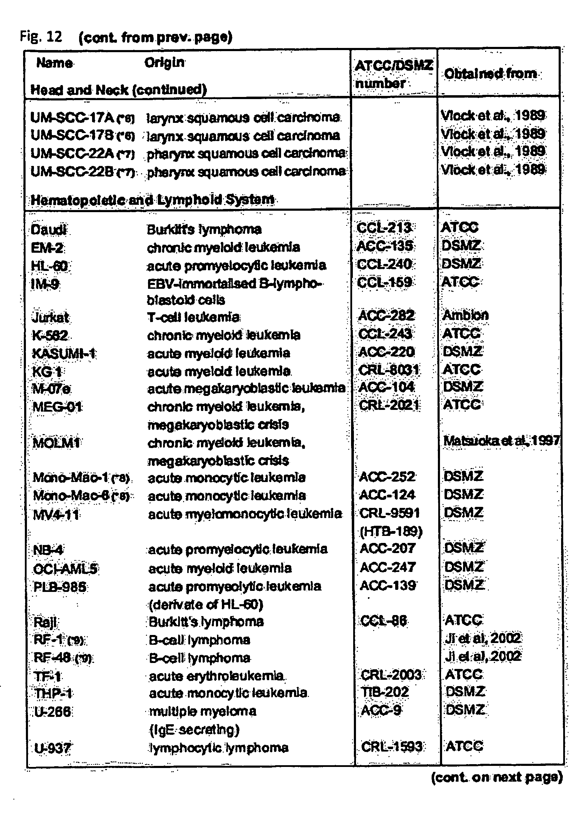

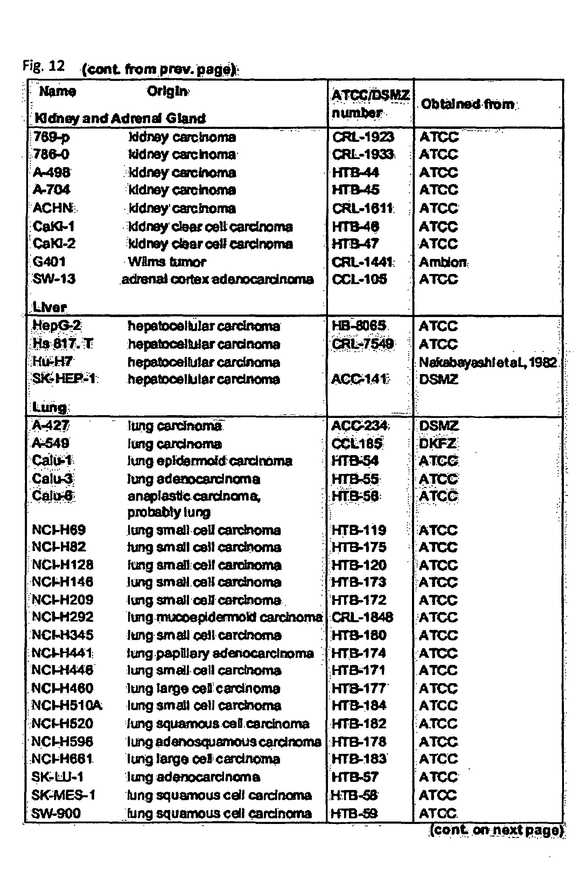

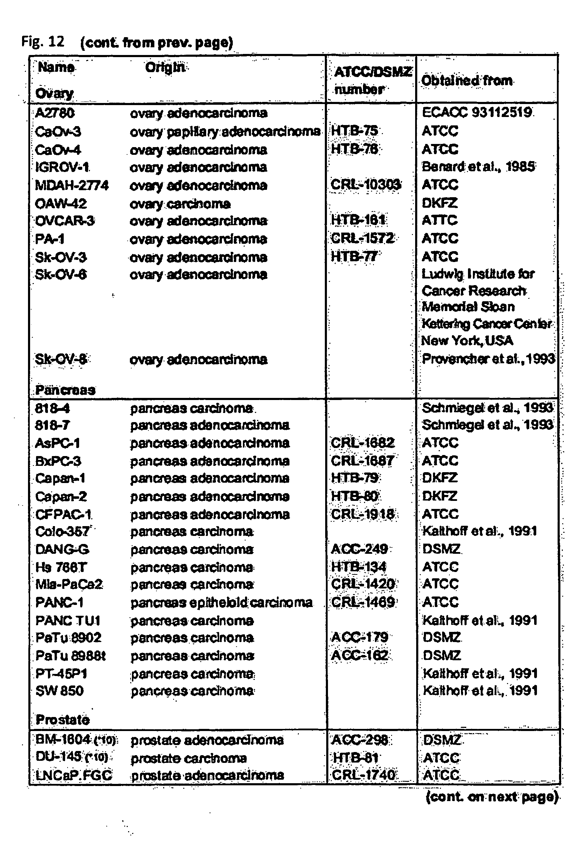

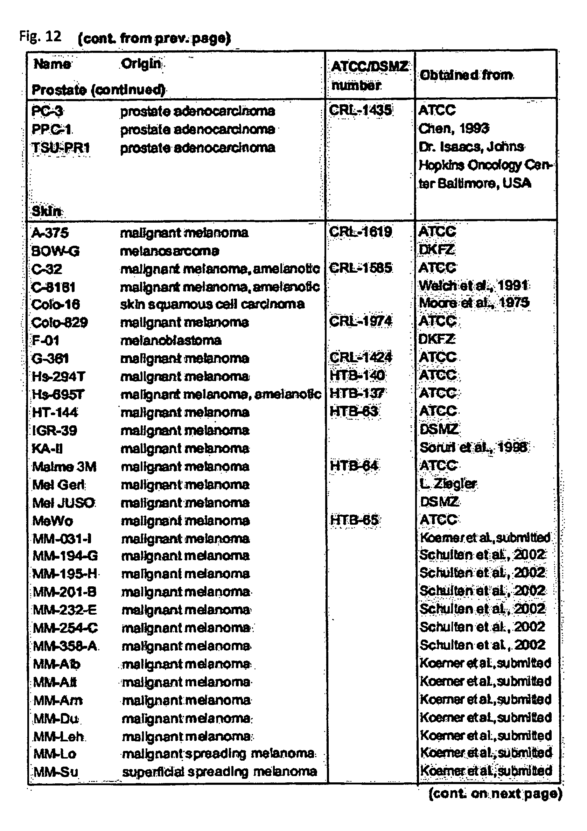

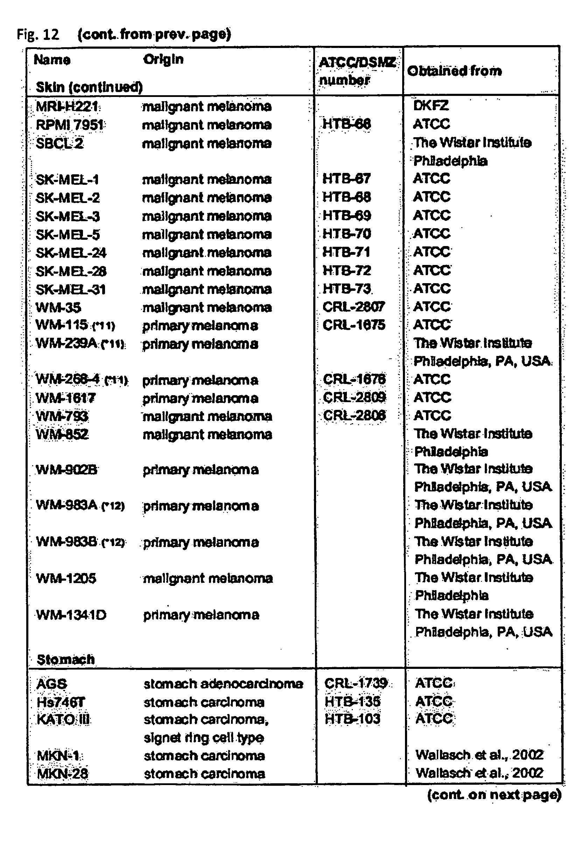

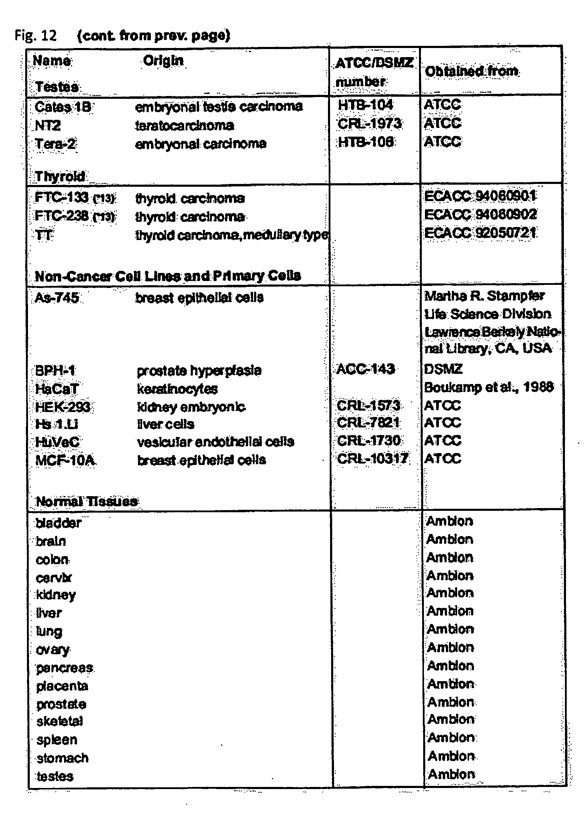

**Fig. 12** depicts tumor cell lines, control cell lines, and tissues from healthy individuals. The name, origin, reference number, and supplier/source of the tumor cell lines, normal cell lines, and tissues from healthy individuals analyzed in the screen are specified. Related tumor cell lines are indicated by parenthesized asterisks and identical numerals. (ATCC: The American Type Culture Collection, Manassas (VA), USA; DKFZ: Tumorbank Deutsches Krebsforschungszentrum, Heidelberg, Germany; DSMZ: German Collection of Microorganisms and Cell Cultures, Braunschweig, Germany; ECACC: The European Collection of Cell Cultures, Porton Down, Salisbury, UK)

**Fig. 13** shows the characterization of each tumor- and control cell line with regard to somatic alterations in the transcripts of PTK genes.

**Fig. 14** depicts the domain localization and tissue distribution of a somatic mutation.

**Fig. 15** shows the primers used for PCR amplification and sequencing of PTK genes.

### DETAILED DESCRIPTION OF THE INVENTION

The present invention relates to mutant kinase polypeptides, nucleic acids encoding such polypeptides, cells containing such nucleic acids, antibodies to such polypeptides, assays utilizing such polypeptides, and methods relating to all of the foregoing. The present invention is based upon the identification of mutant kinase polypeptides involved in human malignancies. The polypeptides and nucleic acids of the invention may be produced using well-known and standard synthesis techniques when given the sequences presented herein.

The term "nucleic acid molecule" as used herein refers to any nucleic acid in any possible configuration, such as single stranded, double stranded or a combination thereof. Nucleic acids include for instance DNA molecules, RNA molecules, analogues of the DNA or RNA generated using nucleotide analogues or using nucleic acid chemistry, locked nucleic acid molecules (LNA), protein nucleic acids molecules (PNA) and tecto-RNA molecules (e.g. Liu, B., et al., J. Am. Chem. Soc. 126, 4076-4077 (2004)). LNA has a modified RNA backbone with a methylene bridge between C4' and O2', providing the respective molecule with a higher duplex stability and nuclease resistance. DNA or RNA may be of genomic or synthetic origin. A respective nucleic acid may furthermore contain non-natural nucleotide analogues and/or be linked to an affinity tag or a label.

Many nucleotide analogues are known and can be used in nucleic acids used in the methods of the invention. A nucleotide analogue is a nucleotide containing a modification at for instance the base, sugar, or phosphate moieties. As an illustrative example, a substitution of 2'-OH residues of siRNA with 2'F, 2'O-Me or 2'H residues is known to improve the *in* vivo stability of the respective RNA. Modifications at the base moiety include natural and synthetic modifications of A, C, G, and T/U, different purine or pyrimidine bases, such as uracil-5-yl, hypoxanthin-9-yl, and 2-aminoadenin-9-yl, as well as non-purine or non-pyrimidine nucleotide bases. Other nucleotide analogues serve as universal bases. Universal bases include 3-nitropyrrole and 5-nitroindole. Universal bases are able to form a base pair with any other base. Base modifications often can be combined with for example a sugar modification, such as for instance 2'-O-methoxyethyl, e.g. to achieve unique properties such as increased duplex stability.

### I. The Nucleic Acids

As mentioned above, the invention also relates to nucleic acid molecules that encode a mutant protein kinase polypeptide. In one embodiment, the mutant protein kinase polypeptide is FGFR4 and the mutant kinase includes FGFR4 Y367C (SEQ ID No. 133). The nucleic acid molecule may be isolated, enriched, or purified.

Also included is a nucleic acid encoding mutant kinase polypeptide fragment of amino acid sequence set forth in SEQ ID No: 133, as long as the mutation or mutated region is retained. In some embodiments, the fragment is at least 10, at least 15, at least 20, at least 30 or at least 35 amino acids long.

By "isolated" in reference to a nucleic acid is meant a polymer of nucleotides conjugated to each other, including DNA and RNA, that is isolated from a natural source or that is synthesized. The isolated nucleic acids of the present invention are not found in a pure or separated state in nature. Use of the term "isolated" indicates that a naturally occurring sequence has been removed from its normal cellular (*i.e.,* chromosomal) environment. Thus, the sequence may be in a cell-free solution or placed in a different cellular environment. The term does not imply that the sequence is the only nucleotide chain present, but that it is essentially free (about 90 - 95% pure at least) of non-nucleotide material naturally associated with it, and thus is distinguished from isolated chromosomes.

By the use of the term "enriched" in reference to nucleic acid is meant that the specific DNA or RNA sequence constitutes a significantly higher fraction (2 - 5 fold) of the total DNA or RNA present in the cells or solution of interest than in normal or diseased cells or in the cells from which the sequence was taken. This could be achieved by reducing the amount of other DNA or RNA present, or by increasing the amount of the specific DNA or RNA sequence, or by a combination of the two. However, it should be noted that being enriched does not imply that there are no other DNA or RNA sequences present, it merely defines that the relative amount of the sequence of interest has been significantly increased.

The term "significant" is used to indicate that the level of increase is useful to the person making such an increase, and generally means an increase relative to other nucleic acids of about at least about 2 fold, such as at least about 5 to about 10 fold or even more. The term does also not imply that there is no DNA or RNA from other sources present. As an illustrative example, another source of DNA may, for example, include a yeast or bacterial genome, or a cloning vector. This term distinguishes from naturally occurring events, such as viral infection, or tumor type growths, in which the level of one mRNA may be naturally increased relative to other species of mRNA. That is, the term is meant to cover only those situations in which a person has intervened to elevate the proportion of the desired nucleic acid.

It is also advantageous for some purposes that a nucleotide sequence be present in purified form. The term "purified" in reference to nucleic acid does not require absolute purity (such as a homogeneous preparation). Instead, it represents an indication that the sequence is relatively more pure than in the natural environment (compared to the natural level this level should be at least 2-5 fold greater, *e.g.,* in terms of mg/ml). Individual clones isolated from a cDNA library may be purified to electrophoretic homogeneity. The claimed DNA molecules obtained from these clones could be obtained directly from total DNA or from total RNA. The cDNA clones are not naturally occurring, rather they are typically obtained via manipulation of a partially purified naturally occurring substance (messenger RNA). The construction of a cDNA library from mRNA involves the creation of a synthetic substance (cDNA) and pure individual cDNA clones can be isolated from the synthetic library by clonal selection of the cells carrying the cDNA library. Thus, the process which includes the construction of a cDNA library from mRNA and isolation of distinct cDNA clones yields an approximately 10⁶-fold purification of the native message. Thus, purification of at least one order of magnitude, including two or three orders, and more, such as four or five orders of magnitude is expressly contemplated.

By a "mutant kinase polypeptide" as used herein is meant a protein kinase polypeptide including a somatic mutation. Such a mutation may be a deletion, insertion or substitution of one or more amino acids. In some embodiments, the term may refer to a contiguous sequence of at least about 50, such as about 100, about 200, or about 300 amino acids set forth in the amino acid sequence of SEQ ID No: 133 or the corresponding full-length amino acid sequence, with the proviso that the desired mutation is included in said amino acid sequence.

In case the mutation leads to a premature stop codon in the nucleotide sequence encoding the mutant kinase polypeptide, the sequence may even be shorter than the above 50 amino acids. The kinase polypeptide can be encoded by a full-length nucleic acid sequence, i.e. the complete coding sequence of the respective gene, or any portion of the full-length nucleic acid sequence, as long as the mutation of the polypeptide is retained.

The amino acid sequence will be substantially similar to the sequence shown in SEQ ID No: 133, or to fragments thereof. A sequence that is substantially similar to the sequence of SEQ ID No: 133 or fragment thereof will in some embodiments have at least about 80, such as at least about 90% identity (in some embodiments at least about 95% or 99-100%) to the sequence of SEQ ID No: 133, with the proviso that the desired mutation is retained.

The kinase polypeptide can be encoded by a full-length nucleic acid sequence, i.e. the complete coding sequence of the respective gene, or any portion of the full-length nucleic acid sequence, as long as the alteration of the polypeptide is retained.

By "identity" is meant a property of sequences that measures their similarity or relationship. Identity is measured by dividing the number of identical residues by the total number of residues and gaps and multiplying the product by 100.

"Gaps" are spaces in an alignment that are the result of additions or deletions of amino acids. Thus, two copies of exactly the same sequence have 100% identity, but sequences that are less highly conserved, and have deletions, additions, or replacements, may have a lower degree of identity. Those skilled in the art will recognize that several computer programs are available for determining sequence identity using standard parameters, for example Blast (Altschul, et al. (1997) Nucleic Acids Res. 25:3389-3402), Blast2 (Altschul, et al. (1990) J. Mol. Biol. 215:403-410), and Smith-Waterman (Smith, et al. (1981) J. Mol. Biol. 147:195-197).

The term "mutated" or "mutant" in reference to a nucleic acid or a polypeptide refers to the exchange, deletion, or insertion of one or more nucleotides or amino acids, respectively, compared to the naturally occurring nucleic acid or polypeptide.

Nucleic acid molecules may be substantially complementary to the above nucleic acid molecules. "Substantially complementary" as used herein refers to the fact that a given nucleic acid molecule is at least 90, at least 95, or 99 or 100% complementary to another nucleic acid. The term "complementary" or "complement" refers to two nucleotides that can form multiple favorable interactions with one another. Such favorable interactions include Watson-Crick base pairing. A nucleotide sequence is the complement of another nucleotide sequence if all of the nucleotides of the first sequence are complementary to all of the nucleotides of the second sequence.

The nucleic acid according to the invention may be isolated from a natural source by cDNA cloning or subtractive hybridization or other routine techniques known to a person skilled in the art. The natural source may be any organism. As an illustrative example, the nucleic acid may be isolated from a mammalian source. It may for example be of human origin. The natural source can include blood, semen, or tissue.

The term "mammalian" refers to any mammal, for example, species such as mice, rats, rabbits, guinea pigs, sheep, and goats, cats, dogs, monkeys, apes, and humans.

The nucleic acid of the invention may also be synthetic, meaning being synthesized by the triester method or by using an automated DNA synthesizer.

For example, it would be appreciated that the nucleic acid moleculementioned herein encoding mutant kinase polypeptide, may further include a vector or promoter effective to initiate transcription in a host cell. The recombinant nucleic acid can alternatively contain a transcriptional initiation region functional in a cell, a sequence complementary to an RNA sequence encoding a kinase polypeptide and a transcriptional termination region functional in a cell.

The nucleic acid may be of recombinant origin, such as a cell or an organism.

The term "vector" relates to a single or double-stranded circular nucleic acid molecule that can be transfected into cells and replicated within or independently of a cell genome. A circular double-stranded nucleic acid molecule can be cut and thereby linearized upon treatment with restriction enzymes. An assortment of nucleic acid vectors, restriction enzymes, and the knowledge of the nucleotide sequences cut by restriction enzymes are readily available to those skilled in the art. A nucleic acid molecule encoding a kinase can be inserted into a vector by cutting the vector with restriction enzymes and ligating the two pieces together.

The term "promoter" refers to nucleic acid sequence needed for gene sequence expression. Promoter regions vary from organism to organism, but are well known to persons skilled in the art for different organisms. For example, in prokaryotes, the promoter region contains both the promoter (which directs the initiation of RNA transcription) as well as the DNA sequences which, when transcribed into RNA, will signal synthesis initiation. Such regions will normally include those 5'-non-coding sequences involved with initiation of transcription and translation, such as the TATA box, capping sequence, CAAT sequence, and the like.

The nucleic acid according to the invention may include, consist essentially of or consist of the nucleotide sequence set forth in SEQ ID No: 402.

Included within the scope of this invention are the functional equivalents of the herein-described isolated nucleic acid molecule. The degeneracy of the genetic code permits substitution of certain codons by other codons that specify the same amino acid and hence would give rise to the same protein. The nucleic acid sequence can vary substantially since, with the exception of methionine and tryptophan, the known amino acids can be coded for by more than one codon. Thus, portions or all of the kinase genes of the invention could be synthesized to give a nucleic acid sequence significantly different from that shown in SEQ ID No: 2. The encoded amino acid sequence thereof would, however, be preserved.

In addition, the nucleic acid sequence may include a nucleotide sequence which results from the addition, deletion or substitution of at least one nucleotide to the 5'-end and/or the 3'-end of the nucleic acid formula shown in SEQ ID No: 402 or a derivative thereof.

Any nucleotide or polynucleotide may be used in this regard, provided that its addition, deletion or substitution does not alter the amino acid sequence of SEQ ID No: 133, which is encoded by the nucleotide sequence. For example, the present invention is intended to include any nucleic acid sequence resulting from the addition of ATG as an initiation codon at the 5'-end of the inventive nucleic acid sequence or its derivative, or from the addition of TTA, TAG or TGA as a termination codon at the 3'-end of the inventive nucleotide sequence or its derivative. Moreover, the nucleic acid molecule of the present invention may, as necessary, have restriction endonuclease recognition sites added to its 5'-end and/or 3'-end.

Such functional alterations of a given nucleic acid sequence afford an opportunity to promote secretion and/or processing of heterologous proteins encoded by foreign nucleic acid sequences fused thereto. All variations of the nucleotide sequence of the kinase genes of the invention and fragments thereof permitted by the genetic code are, therefore, included in this invention.

Further, it is possible to delete codons or to substitute one or more codons with codons other than degenerate codons to produce a structurally modified polypeptide, but one which has substantially the same utility or activity as the polypeptide produced by the unmodified nucleic acid molecule. As recognized in the art, the two polypeptides are functionally equivalent, as are the two nucleic acid molecules that give rise to their production, even though the differences between the nucleic acid molecules are not related to the degeneracy of the genetic code.

### II. Nucleic acid probes, methods, and kits for the detection of mutant kinases.

The nucleic acid molecule of the invention is also useful for the design of hybridization probes to facilitate identification and cloning of mutated kinase polypeptides, the design of PCR probes to facilitate cloning of mutated kinase polypeptides, obtaining antibodies directed against mutated kinase polypeptides, and designing antisense oligonucleotides.

Therefore, the invention is also directed to a nucleic acid probe for the detection of nucleic acid molecules encoding a mutant kinase polypeptide in a sample. The mutant kinase polypeptide is FGFR4, and the mutant kinase polypeptide encoded by said nucleic acid molecule comprises the mutation FGFR4 Y367C (SEQ ID No: 402). The said nucleic acid probe is configured to bind to the FGFR4 having the Y367C mutation and not to a wild type FGF4 or any other FGF4 which does not have the Y367C mutation. The nucleic acid probe contains a nucleotide base sequence that will hybridize to the mutated region of said nucleic acid sequence.

The nucleic acid probe of the invention may include, consist essentially of or consist of nucleotide sequences that will hybridize to a target region in the nucleic acid sequence set forth in SEQ ID No: 402, or a functional equivalent thereof.

The kinase "target region" is the nucleotide base sequence set forth in SEQ ID No: 402 or the corresponding full-length sequences, a functional derivative thereof, or a fragment thereof to which the nucleic acid probe will specifically hybridize, as long as said nucleotide base sequence includes any one of the above indicated mutations or alterations. Specific hybridization indicates that in the presence of other nucleic acids the probe only hybridizes detectably with the target region of the mutant kinase of the invention.

For example, a nucleic acid probe of the present invention may be used to probe a sample or a chromosomal/cDNA library by usual hybridization methods to detect the presence of nucleic acid molecule of the present invention. A chromosomal DNA or cDNA library may be prepared from appropriate cells according to methods well established in the art.

In order to obtain a nucleic acid probe having a nucleotide sequence which corresponds to altered portions of the amino acid sequence of the polypeptide of interest, chemical synthesis can be carried out. The synthesized nucleic acid probes may be first used as primers in a polymerase chain reaction (PCR) carried out in accordance with recognized PCR techniques, essentially according to standard PCR Protocols utilizing the appropriate template, in order to obtain the probes of the present invention.

One skilled in the art will readily be able to design such probes based on the sequence disclosed herein using methods of computer alignment and sequence analysis well known in the art. The hybridization probes of the present invention can be labeled by standard labeling techniques such as with a radiolabel, enzyme label, fluorescent label, biotin-avidin label, chemiluminescence, and the like. After hybridization, the probes may be visualized using known methods.

The nucleic acid probes of the present invention include RNA, as well as DNA probes, such probes being generated using techniques known in the art. The nucleic acid probe may be immobilized on a solid support. Examples of such solid supports include, but are not limited to, plastics such as polycarbonate, complex carbohydrates such as agarose and sepharose, and acrylic resins, such as polyacrylamide and latex beads. Techniques for coupling nucleic acid probes to such solid supports are well known in the art.

The test samples suitable for nucleic acid probing methods of the present invention include, for example, cells or nucleic acid extracts of cells, or biological fluids. The samples used in the above-described methods will vary based on the assay format, the detection method and the nature of the tissues, cells or extracts to be assayed. Methods for preparing nucleic acid extracts of cells are well known in the art and can be readily adapted in order to obtain a sample which is compatible with the method utilized.

One method of detecting the presence of nucleic acid of the invention in a sample includes (a) contacting said sample with the above-described nucleic acid probe under conditions such that hybridization occurs, and (b) detecting the presence of said probe bound to said nucleic acid molecule. One skilled in the art would select the nucleic acid probe according to techniques known in the art as described above. Samples to be tested include but should not be limited to RNA samples of human tissue.

The above method may also utilize a set of the nucleic acid probe of the invention to simultaneously detect the presence of the nucleic acid of the invention in a sample. Such a method may be useful for the diagnosis of proliferative diseases or disorders in a subject and may also be useful to predict the risk of cancer with high predictive accuracy and/or to choose an adequate therapy.

The set of nucleic acid probe utilized in such a method of the invention may be chosen in view of the condition to be detected and may include a nucleic acid probe for all or any subset of the nucleic acid molecules that are implicated by the present invention in the predisposition, development and progression of cancer, including the nucleotide sequences set forth in SEQ ID No: 402. Such a subset may include at least 2, for example at least 5, 7, 10, 12, 16, 20, 30, 40, 50, 60, 70, 80, 90, 100, 110, 120, 150, 200, 250, 300, 350 or any other number, for example all of the above sequence.

For the above method the nucleic acid probe may be bound to or immobilized on a solid support. Said solid support may be a chip, for example a DNA microchip.

A kit for detecting the presence of nucleic acid of the invention in a sample includes at least one container means having disposed therein the above-described nucleic acid probe. The kit may further include other containers that include one or more of the following: wash reagents and reagents capable of detecting the presence of bound nucleic acid probe. Examples of detection reagents include, but are not limited to radiolabeled probes, enzymatic labeled probes (horseradish peroxidase, alkaline phosphatase), and affinity labeled probes (biotin, avidin, or steptavidin).

In detail, a compartmentalized kit includes any kit in which reagents are included in separate containers. Such containers include small glass containers, plastic containers or strips of plastic or paper. Such containers allow the efficient transfer of reagents from one compartment to another compartment such that the samples and reagents are not cross-contaminated and the agents or solutions of each container can be added in a quantitative fashion from one compartment to another. Such containers will include a container which will accept the test sample, a container which contains the probe or primers used in the assay, containers which contain wash reagents (such as phosphate buffered saline, Tris buffers, and the like), and containers which contain the reagents used to detect the hybridized probe, bound antibody, amplified product, or the like. One skilled in the art will readily recognize that the nucleic acid probes described in the present invention can readily be incorporated into one of the established kit formats which are well known in the art.

### III. DNA constructs including a nucleic acid molecule and cells containing these constructs.

The invention further describes a recombinant cell or tissue including a nucleic acid molecule according to the invention, as detailed above.

In such cells, the nucleic acid may be under the control of the genomic regulatory elements, or may be under the control of heterologous regulatory elements including a heterologous promoter.

The term "heterologous" refers to the relationship between two or more nucleic acid or protein sequences that are derived from different sources. For example, a promoter is heterologous with respect to a transcribable polynucleotide sequence if such a combination is not normally found in nature. In addition, a particular sequence may be "heterologous" with respect to a cell or organism in that it encodes a protein or is included in a protein, for example a recombinant protein, that is not normally expressed by the host cell, tissue, or species. Such a heterologous protein accordingly generally is or has been inserted into the respective host cell, tissue, or species. Accordingly, a heterologous promoter is not normally coupled *in vivo* transcriptionally to the coding sequence for the kinase polypeptides.

Therefore, the present invention also relates to a recombinant DNA molecule including, 5' to 3', a promoter effective to initiate transcription in a host cell and the above-described nucleic acid molecule. For example, a recombinant DNA molecule may include a vector and an above-described nucleic acid molecule. A nucleic acid molecule may include a transcriptional region functional in a cell, a sequence complementary to an RNA sequence encoding an amino acid sequence corresponding to the above-described polypeptide, and a transcriptional termination region functional in said cell. The above-described molecules may be isolated and/or purified DNA molecules.

A cell or organism may contain an above-described nucleic acid molecule and thereby is capable of expressing a polypeptide. The polypeptide may be purified from cells which have been altered to express the polypeptide. A cell is said to be "altered to express a desired polypeptide" when the cell, through genetic manipulation, is made to produce a protein which it normally does not produce or which the cell normally produces at lower levels. One skilled in the art can readily adapt procedures for introducing and expressing either genomic, cDNA, or synthetic sequences into either eukaryotic or prokaryotic cells.

A nucleic acid molecule, such as DNA, is said to be "capable of expressing" a polypeptide if it contains nucleotide sequences which contain transcriptional and translational regulatory information and such sequences are "operably linked" to nucleotide sequences which encode the polypeptide. An operable linkage is a linkage in which the regulatory DNA sequences and the DNA sequence sought to be expressed are connected in such a way as to permit gene sequence expression. The precise nature of the regulatory regions needed for gene sequence expression may vary from organism to organism, but shall in general include a promoter region which, in prokaryotes, contains both the promoter (which directs the initiation of RNA transcription) as well as the DNA sequences which, when transcribed into RNA, will signal synthesis initiation. Such regions will normally include those 5'-non-coding sequences involved with initiation of transcription and translation, such as the TATA box, capping sequence, CAAT sequence, and the like.

If desired, the non-coding region 3' to the sequence encoding a mutant kinase may be obtained by the above-described methods. This region may be retained for its transcriptional termination regulatory sequences, such as termination and polyadenylation. Thus, by retaining the 3'-region naturally contiguous to the DNA sequence encoding a kinase, the transcriptional termination signals may be provided. Where the transcriptional termination signals are not satisfactorily functional in the expression host cell, then a 3' region functional in the host cell may be substituted.

Two DNA sequences (such as a promoter region sequence and a sequence encoding a mutant kinase of the invention) are said to be operably linked if the nature of the linkage between the two DNA sequences does not (1) result in the introduction of a frame-shift mutation, (2) interfere with the ability of the promoter region sequence to direct the transcription of a gene sequence encoding a kinase, or (3) interfere with the ability of the gene sequence of a kinase to be transcribed by the promoter region sequence.

Thus, a promoter region would be operably linked to a DNA sequence if the promoter were capable of effecting transcription of that DNA sequence. Thus, to express a gene encoding a mutant kinase, transcriptional and translational signals recognized by an appropriate host are necessary.

A gene encoding a kinase (or a functional derivative thereof) may be expressed in either prokaryotic or eukaryotic cells. Prokaryotic hosts are, generally, very efficient and convenient for the production of recombinant proteins and are, therefore, one type of expression system for the mutant kinase of the present invention. Prokaryotes most frequently are represented by various strains of E. *coli.* However, other microbial strains may also be used, including other bacterial strains.

In prokaryotic systems, plasmid vectors that contain replication sites and control sequences derived from a species compatible with the host may be used. Examples of suitable plasmid vectors may include pBR322, pUC118, pUC119 and the like; suitable phage or bacteriophage vectors may include γgt10, γgt11 and the like; and suitable virus vectors may include pMAM-neo, pKRC and the like. The selected vector may have the capacity to replicate in the selected host cell.

Recognized prokaryotic hosts include bacteria such as E. *coli, Bacillus, Streptomyces, Pseudomonas, Salmonella, Serratia,* and the like. However, under such conditions, the polypeptide will not be glycosylated. The prokaryotic host must be compatible with the replicon and control sequences in the expression plasmid.

To express a kinase (or a functional derivative thereof) in a prokaryotic cell, it is necessary to operably link the sequence encoding the kinase to a functional prokaryotic promoter. Such promoters may be either constitutive or regulatable (*i.e.,* inducible or derepressible). Examples of constitutive promoters include the *int* promoter of bacteriophage λ, the *bla* promoter of the β-lactamase gene sequence of pBR322, and the *cat* promoter of the chloramphenicol acetyl transferase gene sequence of pPR325, and the like. Examples of inducible prokaryotic promoters include the major right and left promoters of bacteriophage λ (P_{L} and P_{R}), the *trp, recA, λacZ, λacI,* and *gal* promoters of E. *coli,* the α-amylase (Ulmanen et al., J. Bacteriol. 162:176-182, 1985) and the ζ-28-specific promoters of *B. subtilis* (Gilman et al., Gene Sequence 32:11-20, 1984), and the promoters of the bacteriophages of *Bacillus,* and *Streptomyces* promoters (Ward et al., Mol. Gen. Genet. 203:468-478, 1986). Prokaryotic promoters are reviewed by Glick (Ind. Microbiot. 1:277-282, 1987), Cenatiempo (Biochimie 68:505-516, 1986), and Gottesman (Ann. Rev. Genet. 18:415-442, 1984).

Proper expression in a prokaryotic cell also requires the presence of a ribosome-binding site upstream of the gene sequence-encoding sequence. Such ribosome-binding sites are disclosed, for example, by Gold et al. (Ann. Rev. Microbiol. 35:365-404, 1981). The selection of control sequences, expression vectors, transformation methods, and the like are dependent on the type of host cell used to express the gene. As used herein, "cell", "cell line", and "cell culture" may be used interchangeably and all such designations include progeny. Thus, the words "transformants" or "transformed cells" include the primary subject cell and cultures derived therefrom, without regard to the number of transfers. It is also understood that all progeny may not be precisely identical in DNA content, due to deliberate or inadvertent mutations. However, as defined, mutant progeny have the same functionality as that of the originally transformed cell.

Host cells which may be used in the expression systems are not strictly limited, provided that they are suitable for use in the expression of the kinase polypeptide of interest. Suitable hosts may often include eukaryotic cells. Examples of eukaryotic hosts include, but are not limited to, yeast, fungi, insect cells, mammalian cells either *in vivo,* or in tissue culture. Mammalian cells which may be useful as hosts include for example HeLa cells, cells of fibroblast origin such as VERO or CHO-K1, or cells of lymphoid origin and their derivatives. For example, the mammalian host cells include any, including all human cancer cell lines.

Another suitable host is an insect cell, for example the *Drosophila* larvae. Using insect cells as hosts, the *Drosophila* alcohol dehydrogenase promoter can be used (Rubin, Science 240:1453-1459, 1988). Alternatively, baculovirus vectors can be engineered to express large amounts of kinases in insect cells (Jasny, Science 238:1653, 1987).

Any of a series of yeast expression systems can be utilized which incorporate promoter and termination elements from the actively expressed sequences coding for glycolytic enzymes that are produced in large quantities when yeast are grown in mediums rich in glucose. Known glycolytic gene sequences can also provide very efficient transcriptional control signals. Yeast provides substantial advantages in that it can also carry out post-translational modifications. A number of recombinant DNA strategies exists utilizing strong promoter sequences and high copy number plasmids, which can be utilized for production of the desired proteins in yeast. Yeast recognizes leader sequences on cloned mammalian genes and secretes peptides bearing leader sequences (*i.e.,* pre-peptides). Several possible vector systems are available for the expression of kinases in a mammalian host.

A wide variety of transcriptional and translational regulatory sequences may be employed, depending upon the nature of the host. The transcriptional and translational regulatory signals may be derived from viral sources, such as adenovirus, bovine papilloma virus, cytomegalovirus, simian virus, or the like, where the regulatory signals are associated with a particular gene sequence which has a high level of expression. Alternatively, promoters from mammalian expression products, such as actin, collagen, myosin, and the like, may be employed. Transcriptional initiation regulatory signals may be selected which allow for repression or activation, so that expression of the gene sequences can be modulated. Of interest are regulatory signals which are temperature-sensitive so that by varying the temperature, expression can be repressed or initiated, or are subject to chemical (such as metabolite) regulation.

Expression of mutant kinases in eukaryotic hosts requires the use of eukaryotic regulatory regions. Such regions will, in general, include a promoter region sufficient to direct the initiation of RNA synthesis. Examples of a suitable eukaryotic promoter include, but are not limited to, the promoter of the mouse metallothionein I gene sequence (Hamer et al., J. Mol. Appl. Gen. 1:273-288, 1982); the TK promoter of Herpes virus (McKnight, Cell 31:355-365, 1982); the SV40 early promoter (Benoist et al., Nature 290:304-31, 1981); and the yeast gal4 gene sequence promoter (Johnston et al., Proc. Natl. Acad. Sci. (USA) 79:6971-6975, 1982; Silver et al., Proc. Natl. Acad. Sci. (USA) 81:5951-5955, 1984).

Translation of eukaryotic mRNA is initiated at the codon which encodes the first methionine. For this reason, it may be desired to ensure that the linkage between a eukaryotic promoter and a DNA sequence which encodes a kinase of the invention (or a functional derivative thereof) does not contain any intervening codons which are capable of encoding a methionine (*i.e.,* AUG). The presence of such codons results either in the formation of a fusion protein (if the AUG codon is in the same reading frame as the kinase of the invention coding sequence) or a frame-shift mutation (if the AUG codon is not in the same reading frame as the kinase of the invention coding sequence).

A nucleic acid molecule encoding a kinase and an operably linked promoter may be introduced into a recipient prokaryotic or eukaryotic cell either as a nonreplicating DNA or RNA molecule, which may either be a linear molecule ora closed covalent circular molecule. Since such molecules are incapable of autonomous replication, the expression of the gene may occur through the transient expression of the introduced sequence. Alternatively, permanent expression may occur through the integration of the introduced DNA sequence into the host chromosome.

A vector may be employed which is capable of integrating the desired gene sequences into the host cell chromosome. Cells which have stably integrated the introduced DNA into their chromosomes can be selected by also introducing one or more markers which allow for selection of host cells which contain the expression vector. The marker may provide for prototrophy to an auxotrophic host, biocide resistance, *e.g.,* antibiotics, or heavy metals, such as copper, or the like. The selectable marker gene sequence can either be directly linked to the DNA gene sequences to be expressed, or introduced into the same cell by co-transfection. Additional elements may also be needed for optimal synthesis of mRNA. These elements may include splice signals, as well as transcription promoters, enhancers, and termination signals. cDNA expression vectors incorporating such elements include those described by Okayama (Mol. Cell. Biol. 3:280, 1983).

The nucleic acid molecule can be incorporated into a plasmid or viral vector capable of autonomous replication in the recipient host. Any of a wide variety of vectors may be employed for this purpose. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

An illustrative example of a prokaryotic vector is a plasmid, such as a plasmid capable of replication in *E. coli* (such as, for example, pBR322, ColE1, pSC101, pACYC 184, πVX). Bacillus plasmids include pC194, pC221, pT127, and the like. Suitable *Streptomyces* plasmids include p1J101 (Kendall et al., J. Bacteriol. 169:4177-4183, 1987), and streptomyces bacteriophages such as φC31. *Pseudomonas* plasmids are reviewed by John et al. (Rev. Infect. Dis. 8:693-704, 1986), and Izaki (Jpn. J. Bacteriol. 33:729-742, 1978).

Examples of an eukaryotic plasmid include, but are not limited to, BPV, vaccinia, SV40, 2-micron circle, and the like, or their derivatives. Such plasmids are well known in the art (e.g. Broach, Cell 28:203-204, 1982; Bollon et al., J.Clin. Hematol. Oncol. 10:39-48, 1980).

Once the vector or nucleic acid molecule containing the construct(s) has been prepared for expression, the DNA construct(s) may be introduced into an appropriate host cell by any of a variety of suitable means, i.e., transformation, transfection, conjugation, protoplast fusion, electroporation, particle gun technology, calcium phosphate-precipitation, direct microinjection, and the like. After the introduction of the vector, recipient cells are grown in a selective medium, which selects for the growth of vector-containing cells. Expression of the cloned gene(s) results in the production of a kinase, or fragments thereof. This can take place in the transformed cells as such, or following the induction of these cells to differentiate. A variety of incubation conditions can be used to form the peptide of the present invention. It may be desired to use conditions thatmimic physiological conditions.

The term "transfecting" defines a number of methods to insert a nucleic acid vector or other nucleic acid molecules into a cellular organism. These methods involve a variety of techniques, such as treating the cells with high concentrations of salt, an electric field, detergent, or DMSO to render the outer membrane or wall of the cells permeable to nucleic acid molecules of interest or use of various viral transduction strategies.

### IV. Proteins

The mutant kinase polypeptide of the invention is FGFR4 and includes the mutation FGFR4 Y367C and may be isolated, enriched or purified.

By "isolated" in reference to a polypeptide is meant a polymer of amino acids (2 or more amino acids) conjugated to each other, including polypeptides that are isolated from a natural source or that are synthesized. The isolated polypeptides of the present invention are unique in the sense that they are not found in a pure or separated state in nature. Use of the term "isolated" indicates that a naturally occurring sequence has been removed from its normal cellular environment. Thus, the sequence may be in a cell-free solution or placed in a different cellular environment. The term does not imply that the sequence is the only amino acid chain present, but that it is essentially free (about 90 - 95% pure at least) of non-amino acid material naturally associated with it.

By the use of the term "enriched" in reference to a polypeptide is meant that the specific amino acid sequence constitutes a significantly higher fraction (2 - 5 fold) of the total amino acid sequences present in the cells or solution of interest than in normal or diseased cells or in the cells from which the sequence was taken. This could be caused by preferential reduction in the amount of other amino acid sequences present, or by a preferential increase in the amount of the specific amino acid sequence of interest, or by a combination of the two. However, it should be noted that enriched does not imply that there are no other amino acid sequences present. The term merely defines that the relative amount of the sequence of interest has been significantly increased. The term significant here is used to indicate that the level of increase is useful to the person making such an increase, and generally means an increase relative to other amino acid sequences of about at least 2-fold, for example at least about 5- to 10-fold or even more. The term also does not imply that there is no amino acid sequence from other sources. The other source of amino acid sequences may, for example, include amino acid sequence encoded by a yeast or bacterial genome, or a cloning vector. The term is meant to cover only those situations in which man has intervened to increase the proportion of the desired amino acid sequence.

It is also advantageous for some purposes that an amino acid sequence be in purified form. The term "purified" in reference to a polypeptide does not require absolute purity (such as a homogeneous preparation); instead, it represents an indication that the sequence is relatively purer than in the natural environment. Compared to the natural level this level should be at least 2-5 fold greater (e.g., in terms of mg/ml). Purification of at least one order of magnitude, such as about two or three orders, including for example about four-or five orders of magnitude is expressly contemplated. It may be desired to obtain the substance at least essentially free of contamination at a functionally significant level, for example about 90%, about 95%, or 99% pure.

Explicitly falling within the scope of the present invention are fragments of mutant kinase polypeptide with an amino acid sequence set forth in SEQ ID No: 133, or the corresponding full-length amino acid sequence thereof, as long as said fragments include a respective mutation (nucleotides: A1256G; the nucleic acid sequence ("nt") 2). The mutant kinase polypeptide fragments contain at least 30, 35, 40, 45, 50, 60, 100, 200, or 300 contiguous amino acids of SEQ ID No: 133, provided that the mutation of interest is included in said protein fragment.

In case a mutation leads to a premature stop codon, the mutated kinase may be even shorter than 30 amino acids. However, such mutants are also considered to fall within the scope of the present invention.

By "fragment" in reference to a polypeptide is meant any amino acid sequence present in a kinase polypeptide, as long as it is shorter than the full length sequence.

A variety of methodologies known in the art can be utilized to obtain the polypeptide of the present invention. The polypeptide may be purified from tissues or cells that naturally produce the polypeptide. Alternatively, the above-described isolated nucleic acid fragments could be used to express the recombinant kinase polypeptide of the invention in any organism.

By "recombinant kinase polypeptide" is meant a polypeptide produced by recombinant DNA techniques such that it is distinct from a naturally occurring polypeptide either in its location (*e.g.,* present in a different cell or tissue than found in nature), purity or structure. Generally, such a recombinant polypeptide will be present in a cell in an amount different from that normally observed in nature.

Any eukaryotic organism can be used as a source for the polypeptides of the invention, as long as the source organism naturally contains such polypeptides. As used herein, "source organism" refers to the original organism from which the amino acid sequence of the subunit is derived, regardless of the organism the subunit is expressed in and ultimately isolated from.

For example, the polypeptide may be synthesized using an automated polypeptide synthesizer.

One skilled in the art can readily follow known methods for isolating proteins in order to obtain the polypeptides free of natural contaminants. These include, but are not limited to: size-exclusion chromatography, HPLC, ion-exchange chromatography, and immuno-affinity chromatography.

### V. Antibodies, methods of their use and kits for the detection of mutant kinase. polypeptides

Antibodies having specific binding affinity only for a mutant kinase polypeptide, or domain or fragment thereof, with the mutant kinase polypeptide FGFR4 and including the mutation FGFR4 Y367C are provided.

By "specific binding affinity" is meant that the antibody binds to the target kinase polypeptide with greater affinity than it binds to other polypeptides under specified conditions. Antibodies or antibody fragments are polypeptides that contain regions that can bind other polypeptides. The term "specific binding affinity" describes an antibody that binds to a mutant kinase polypeptide with significantly greater affinity than it binds to other polypeptides, e.g. the native kinase, under specified conditions.

The term "polyclonal" refers to antibodies that are heterogenous populations of antibody molecules derived from the sera of animals immunized with an antigen or an antigenic functional derivative thereof. For the production of polyclonal antibodies, various host animals may be immunized by injection with the antigen. Various adjuvants may be used to increase the immunological response, depending on the host species.

"Monoclonal antibodies" are substantially homogenous populations of antibodies to a particular antigen. They may be obtained by any technique which provides for the production of antibody molecules by continuous cell lines in culture. Monoclonal antibodies may be obtained by methods well known to those skilled in the art (see for example, Köhler et al., Nature 256:495-497 (1975), and U.S. Patent No. 4,376,110).

The term "antibody fragment" refers to a portion of an antibody, often the hypervariable region and portions of the surrounding heavy and light chains that displays specific binding affinity for a particular molecule. A hypervariable region is a portion of an antibody that physically binds to the polypeptide target.

The term "domain" refers to a region of a polypeptide which contains a particular function. For instance, N-terminal or C-terminal domains of signal transduction proteins can serve functions including, but not limited to, binding molecules that localize the signal transduction molecule to different regions of the cell or binding other signaling molecules directly responsible for propagating a particular cellular signal. Some domains can be expressed separately from the rest of the protein and function by themselves, while others must remain part of the intact protein to retain function. The latter are termed functional regions of proteins and also relate to domains.

An antibody may be isolated by comparing its binding affinity to a mutant kinase with its binding affinity to other polypeptides. Those which bind selectively to a mutant kinase may be chosen for use in methods requiring a distinction between a kinase of interest and other polypeptides. Such methods could include, but should not be limited to, the analysis of altered kinase expression in tissue containing other polypeptides.

The mutant kinases can be used in a variety of procedures and methods, such as for the generation of antibodies and for use in identifying pharmaceutical compositions. One skilled in the art will recognize that if an antibody is desired, a mutant kinase could be generated as described herein and used as an immunogen. The antibodies include monoclonal and polyclonal antibodies, as well fragments of these antibodies, and humanized forms. Humanized forms of the antibodies may be generated using one of the procedures known in the art such as chimerization or CDR grafting.

In general, techniques for preparing monoclonal antibodies and hybridomas are well known in the art. Any animal (mouse, rabbit, and the like) which is known to produce antibodies can be immunized with the selected polypeptide. Methods for immunization are well known in the art. Such methods include subcutaneous or intraperitoneal injection of the polypeptide. One skilled in the art will recognize that the amount of polypeptide used for immunization will vary based on the animal which is immunized, the antigenicity of the polypeptide and the site of injection.

The polypeptide may be modified or administered in an adjuvant in order to increase the peptide antigenicity. Methods of increasing the antigenicity of a polypeptide are well known in the art. Such procedures include coupling the antigen with a heterologous protein (such as globulin or β-galactosidase) or through the inclusion of an adjuvant during immunization.

For monoclonal antibodies, spleen cells from the immunized animals are removed, fused with myeloma cells, such as SP2/0-Ag14 myeloma cells, and allowed to become monoclonal antibody producing hybridoma cells. Any one of a number of methods well known in the art can be used to identify the hybridoma cell which produces an antibody with the desired characteristics. These include screening the hybridomas with an ELISA assay, western blot analysis, or radioimmunoassay (Lutz et al., Exp. Cell Res. 175:109-124, 1988). Hybridomas secreting the desired antibodies are cloned and the class and subclass are determined using procedures known in the art.

For polyclonal antibodies, antibody-containing antisera is isolated from the immunized animal and is screened for the presence of antibodies with the desired specificity using one of the above-described procedures. The above-described antibodies may be detectably labeled. Antibodies can be detectably labeled through the use of radioisotopes, affinity labels (such as biotin, avidin, and the like), enzymatic labels (such as horse radish peroxidase, alkaline phosphatase, and the like) fluorescent labels (such as FITC or rhodamine, and the like), paramagnetic atoms, and the like. Procedures for accomplishing such labeling are well-known in the art, for example, *see* Stemberger et al., J. Histochem. Cytochem. 18:315, 1970; Bayer et al., Meth. Enzym. 62:308-, 1979; Engval et al., Immunol. 109:129-, 1972; Goding, J. Immunol. Meth. 13:215-, 1976. The labeled antibodies of the present invention can be used f*o*r *in vitro, in vivo,* and *in situ* assays to identify cells or tissues which express a specific peptide.

The above-described antibodies may also be immobilized on a solid support. Examples of such solid supports include plastics such as polycarbonate, complex carbohydrates such as agarose and sepharose, acrylic resins and such as polyacrylamide and latex beads. Techniques for coupling antibodies to such solid supports are well known in the art. The immobilized antibodies of the present invention can be used for *in vitro, in vivo,* and *in situ* assays.as well as in immunochromatography.

A method of detecting a mutant kinase polypeptide in a sample, may include: (a) contacting the sample with an above-described antibody, under conditions such that immunocomplexes form, and (b) detecting the presence of said antibody bound to the polypeptide. In detail, the method include incubating a test sample with one or more of the antibodies and assaying whether the antibody binds to the test sample. The presence of a mutant kinase in a sample may indicate disease.

Conditions for incubating an antibody with a test sample vary. Incubation conditions depend on the format employed in the assay, the detection methods employed, and the type and nature of the antibody used in the assay. One skilled in the art will recognize that any one of the commonly available immunological assay formats (such as radioimmunoassays, enzyme-linked immunosorbent assays, diffusion based Ouchterlony, or rocket immunofluorescent assays) can readily be adapted to employ the antibodies of the present invention..

The immunological assay test samples include cells, protein or membrane extracts of cells, or biological fluids such as blood, serum, plasma, or urine. The test samples used in the above-described method will vary based on the assay format, nature of the detection method and the tissues, cells or extracts used as the sample to be assayed.

Methods for preparing protein extracts or membrane extracts of cells are well known in the art and can be readily be adapted in order to obtain a sample which is testable with the system utilized.

Diagnostic kits for performing such methods may contain all the necessary reagents to carry out the previously described methods of detection. The kit may include antibodies or antibody fragments specific for the mutant kinase as well as a conjugate of a binding partner of the antibodies or the antibodies themselves. Diagnostic kits for performing such methods may be constructed to include a first container containing the antibody and a second container having a conjugate of a binding partner of the antibody and a label, such as, for example, a radioisotope. For example, the kit may further include one or more other containers including one or more of the following: wash reagents and reagents capable of detecting the presence of bound antibodies.

Examples of detection reagents include, but are not limited to, labeled secondary antibodies, or in the alternative, if the primary antibody is labeled, the chromophoric, enzymatic, or antibody binding reagents which are capable of reacting with the labeled antibody. The compartmentalized kit may be as described above for nucleic acid probe kits. One skilled in the art will readily recognize that the antibodies can readily be incorporated into one of the established kit formats which are well known in the art.

### VI. Exemplary protein kinase mutants and their use

In one aspect the present invention relates to an isolated, enriched, or purified nucleic acid molecule that encodes a mutant of a protein kinase polypeptide with the protein kinase polypeptide being FGFR4.

FGFR4 is a member of the fibroblast growth factor transmembrane receptor family. FGF-receptors stimulate growth of many cell types and are inter alia involved in tissue repair, wound healing and angiogenesis. The FGF receptors include a vatiety of splice variants. Each receptor and receptor splice variant is activated by a unique set of fibroblast growth factors (see Powers, C.M., et al., Endocr. Relat. Cancer 7:165-197 (2000)). Cellular signaling pathways by FGF receptors have recently been reviewed by Eswarakumar et al (Cytokine & Growth Factor Reviews 16, 139-149 (2005)). Fibroblast growth factor receptors are known to activate the Ras-MAPK, the PLCγ-PKC, the PI3K-Akt and the p38 MAPK pathways. They are also known to play a role in tumor development and progression. FGFR4 is overexpressed in clinical prostate cancer and suppression of FGFR4 expression blocks prostate cancer proliferation (Sahadevan, K., et al. J. Pathology 213: 82-90 (2007)).

In one aspect the invention relates to a nucleic acid molecule that encodes FGFR4 Y367C. This nucleic acid accordingly encodes an FGFR protein that has at position 367 Cysteine rather than Tyrosine, as would be the case for the wild type protein. This amino acid is highly conserved throughout the FGFR family and located within the extracellular domain of the receptor. The amino acid exchange may facilitate receptor dimerization, thereby augmenting receptor activation and resulting in a basal receptor activation.

A respective method may include identifying the amino acid at position 367 of the expressed protein kinase FGFR4. The presence of Cysteine at position 367 indicates an increased predisposition to turn tumorigenic, including to transform into a cancer cell.

A method of identifying a cell that has a predisposition to turn tumorigenic, including to transform into a cancer cell, may be used in combination with any other diagnostic or prognostic method, e.g. a method of cancer prognosis or diagnosis. As an illustrative example, where the cell of interest is a liver cell, the level of the marker protein alpha-fetoprotein may be determined (see above and below). The method may also be combined with any other desired method. For example, a method may be combined with detecting the expression of one or more marker genes of the respective tissue type of the cell in question. Any such combination may also be carried out with a respective method of identifying a cell that is resistant to apoptosis inducing reagents (see below).

### VII. Identification of comnounds modulating mutant kinase activity

Encompassed by the instant disclosure are also methods for the identification of a compound capable of modulating the activity of a mutant protein kinase polypeptide of the invention. Said mutant kinase polypeptide is as defined above.

The term "kinase activity", as used herein, may relate to the catalytic activity of a kinase and thus define the rate at which a kinase catalytic domain phosphorylates a substrate. Catalytic activity can be measured, for example, by determining the amount of a substrate converted to a phosphorylated product as a function of time. Catalytic activity can be measured by methods by holding time constant and determining the concentration of a phosphorylated substrate after a fixed period of time. Phosphorylation of a substrate occurs at the active site of a protein kinase. The active site is normally a cavity in which the substrate binds to the protein kinase and is phosphorylated. The term "kinase activity" may also relate to the binding of a kinase to a natural binding partner that may, but must not include phosphorylation.

The term "kinase catalytic domain" refers to a region of the protein kinase that is typically 25-300 amino acids long and is responsible for carrying out the phosphate transfer reaction from a high-energy phosphate donor molecule such as ATP or GTP to itself (autophosphorylation) or to other proteins (heterologous phosphorylation). The catalytic domain of protein kinases is made up of 12 subdomains that contain highly conserved amino acid residues, and are responsible for proper polypeptide folding and for catalysis. The catalytic domain can be identified following, for example, a Smith-Waterman alignment of the protein sequence against the non-redundant protein database.

The term "substrate" as used herein refers to a molecule phosphorylated by a kinase. Kinases phosphorylate substrates on serine/threonine or tyrosine amino acids. The molecule may be another protein or a polypeptide.

By "functional" domain is meant any region of the polypeptide that may play a regulatory or catalytic role as predicted from amino acid sequence homology to other proteins or by the presence of amino acid sequences that may give rise to specific structural conformations (*i.e.* coiled-coils).

The term "modulates" refers to the ability of a compound to alter the function of a mutated kinase of the invention. A modulator typically activates or inhibits the activity of a mutated kinase of the invention depending on the concentration of the compound exposed to the kinase. The modulator may inhibit the activity of a mutated kinase of the invention. The compound may be capable of differentiating between a native and mutant form and/or between the distinct variants of said kinase.

The term "activates" refers to increasing the cellular activity of the kinase. The term "inhibits" refers to decreasing the cellular activity of the kinase. Kinase activity may be the interaction with a natural binding partner, including phosphorylation.

The term "modulates" also refers to altering the function of mutant kinase of the invention by increasing or decreasing the probability that a complex forms between the kinase and a natural binding partner. A modulator may increase or decrease the probability that such a complex forms between the kinase and the natural binding partner depending on the concentration of the compound exposed to the kinase.In some embodiments the modulator decreases the probability that a complex forms between the kinase and the natural binding partner.

The term "complex" refers to an assembly of at least two molecules bound to one another. Signal transduction complexes often contain at least two protein molecules bound to one another. For instance, a protein tyrosine receptor protein kinase, GRB2, SOS, RAF, and RAS assemble to form a signal transduction complex in response to a mitogenic ligand.

The term "natural biding partner" refers to polypeptides, lipids, small molecules, or nucleic acids that bind to kinases in cells. The natural binding partner may be a nucleotide, such as ATP or GTP or an analogue thereof, or a protein. It may be a protein that is involved in signal transduction pathways. A change in the interaction between a kinase and a natural binding partner can manifest itself as an increased or decreased probability that the interaction forms, or an increased or decreased concentration of kinase/natural binding partner complex.

Such a method includes the steps of: (a) contacting a mutant kinase polypeptide with a test substance; (b) measuring the activity of said polypeptide; and (c) determining whether said substance modulates the activity of said polypeptide.

As a further illustrative example, in some embodiments of the present method of the invention the expression of the respective protein kinase is reduced by means of a non-coding nucleic acid molecule, such as for example an aptamer or a Spiegelmer® (described in WO 01/92655). A non-coding nucleic acid molecule may also be an nc-RNA molecule (see e.g. Costa, FF, Gene (2005), 357, 83-94 for an introduction on natural nc-RNA molecules). Examples of nc-RNA molecules include, but are not limited to, an anti-sense-RNA molecule, an L-RNA Spiegelmer®, a silencer-RNA molecule (such as the double-stranded Neuron Restrictive Silencer Element), a micro RNA (miRNA) molecule, a short hairpin RNA (shRNA) molecule, a small interfering RNA (siRNA) molecule, a repeat-associated small interfering RNA (rasiRNA) molecule or an RNA that interacts with Piwi proteins (piRNA) (for a brief review see e.g. Lin, H., Science (2007) 316, 397).

The use of small interfering RNAs has become a tool to "knock down" specific genes. An overview on the differences between the use of synthetic small organic compounds and RNAi has been given by Weiss et al. (Nature Chem. Biol. 3, 12, 739-744 (2007)). Small interfering RNA makes use of gene silencing or gene suppression through RNA interference (RNAi), which occurs at the posttranscriptional level and involves mRNA degradation. RNA interference represents a cellular mechanism that protects the genome. SiRNA molecules mediate the degradation of their complementary RNA by association of the siRNA with a multiple enzyme complex to form what is called the RNA-induced silencing Complex (RISC). The siRNA becomes part of RISC and is targeted to the complementary RNA species which is then cleaved. This leads to the loss of expression of the respective gene (for a brief overview see Zamore, PD, & Haley, B, Science 309, 1519-1524 [2005]). This technique has for example been applied to silencing parasitic DNA sequences, such as the cleavage of HIV RNA, as disclosed in US patent application 2005/0191618.

For example, such a siRNA may include an in vitro or in vivo synthesized molecule of 10 to 35 nucleotides, in other examples 15 to 25 nucleotides. A respective si-RNA molecule may be directly synthesized within a cell of interest (including a cell that is part of a microorganism and an animal). It may also be introduced into a respective cell and/or delivered thereto. An illustrative example of delivering a siRNA molecule into selected cells in vivo is its non-covalent binding to a fusion protein of a heavy-chain antibody fragment (Fab) and the nucleic acid binding protein protamin (Song, E. et al., Nature Biotech. 23, 6, 709-717 [2005]). The siRNA molecules may be used to induce a degradation of mRNA molecules encoding one or more protein kinases of interest.

### VIII. Methods of use of the molecules

A method may be useful for the detection of a mutant kinase polypeptide in a sample as a diagnostic tool for diseases or disorders. Such a method may include the steps of: (a) contacting the sample with a nucleic acid probe which hybridizes under hybridization assay conditions to a target region of a nucleic acid encoding a mutant kinase polypeptide or the complement thereof; and (b) detecting the presence or amount of the probe:target region hybrid as an indication of the disease or disorder. The mutant kinase polypeptide may be as defined above.

The disease or disorder involving a kinase mutant may be a proliferative disease or disorder, for example cancer.

The nucleic acid probe hybridizes to a mutant kinase target region that encodes at least about 10, about 15, about 20, about 30, about 40, about 50, about 75, about 100, about 150, about 200, about 250 about 300 or about 350 contiguous amino acids of the sequence set forth in SEQ ID NO: 133, or the corresponding full-length amino acid sequence, or a functional derivative thereof, with the proviso that the target region includes the respective mutation set forth in Figure 32. Hybridization conditions should be such that hybridization occurs only with the kinase genes in the presence of other nucleic acid molecules. Under stringent hybridization conditions only highly complementary nucleic acid sequences hybridize. It may be desired to use conditions that prevent hybridization of nucleic acids that have one or more mismatches in a sequence of about 20 contiguous nucleotides.

The diseases that may be diagnosed by detection of a kinase nucleic acid in a sample may include a cancer. The test samples suitable for nucleic acid probing methods of the present invention include, for example, cells or nucleic acid extracts of cells, or biological fluids. The samples used in the above-described methods will vary based on the assay format, the detection method and the nature of the tissues, cells or extracts to be assayed. Methods for preparing nucleic acid extracts of cells are well-known in the art and can be readily adapted in order to obtain a sample that is compatible with the method utilized.

In order that the invention may be readily understood and put into practical effect, particular embodiments will now be described by way of the following non-limiting examples.

### EXEMPLARY EMBODIMENTS

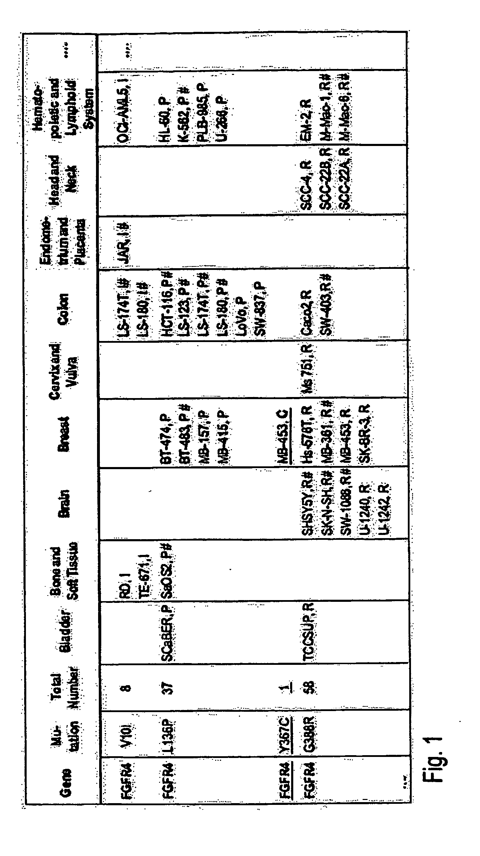

**Fig. 1** depicts a characterization of a protein tyrosine kinase gene with regard to genetic alteration detected in the transcripts of 276 tumor cell lines and control samples. As exemplified here for FGFR4 Y367C, the spectrum of identified genetic alteration (i.e., somatic mutations) and the corresponding pattern of affected tumor cell line or control sample was determined for the tyrosine kinase gene (MB-453, C).

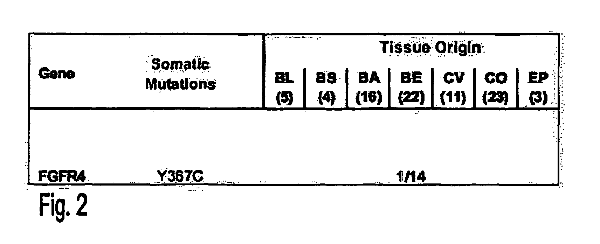

**Fig. 2** shows for the somatic mutation Y367C, the tissue distribution (BE: breast and PR: prostate) was determined. Paired numerals indicate the number of mutated and expression-positive cell lines within a given tumor type.

**Fig. 3B** shows an illustration of sequence comparison of FGFR1-4. For FGFR1-4, the general domain organization (middle) and sequence comparisons of the linker region connecting the IG-D2- and IG-D3-domain (top) as well as a part of the extracellular juxtamembrane region (bottom) are illustrated. Genetic alterations identified in our cell line screen are illustrated below, known sequence variants are depicted above the graphical representation of the domain structure. Polymorphisms are underlined, the remaining marked positions are somatic mutations. Numbers in parenthesis indicate the number of affected non-related cell lines. (SH2: Src Homolgy 2 Domain; TK: Tyrosine Kinase Domain; S: Signal Peptide; TM: Transmembrane Domain; IG: Immunoglobulin-Like Domain).

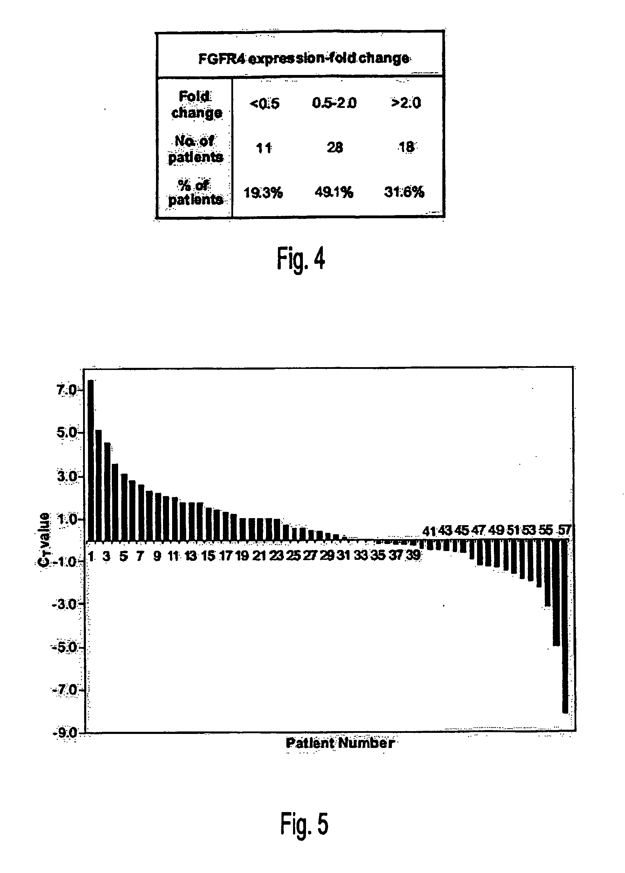

FGFR4 transcript is overexpressed (> 2-fold) in 1/3 of hepatocellular carcinoma (HCC) patients (n= 57) in the tumor vs. the adjacent normal tissue as determined by real-time PCR **(****Fig. 4, Fig. 5****).** The threshold cycle (Ct) value shown in Fig. 5 is scored as the cycle number where the fluorescence level crosses a predefined threshold value. The Cₜ value assigned to a particular sample thus reflects the point during the reaction at which a sufficient number of amplicons have accumulated, in that well, to be at a statistically significant point above the baseline. A lower Cₜ value accordingly reflects a higher relative gene expression.

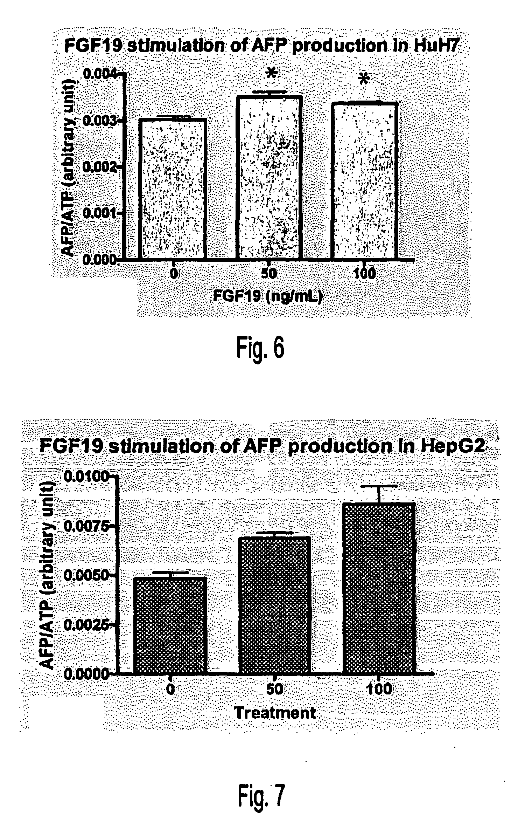

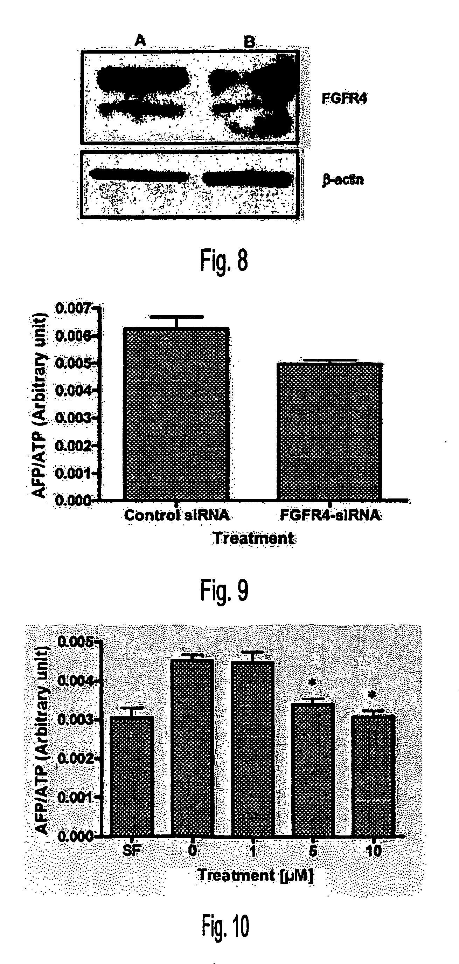

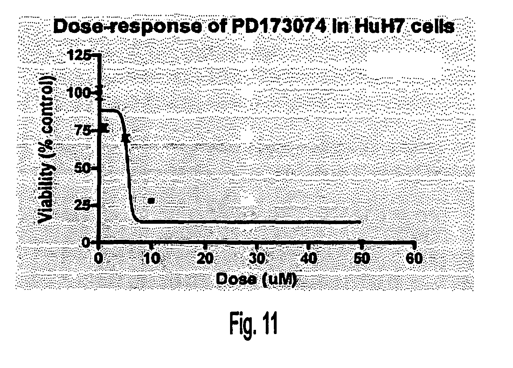

Subsequent *in vitro* investigations revealed that stimulation of FGFR4 in HCC cell lines using a specific ligand, FGF19, elevated AFP production by the cells (Fig. 6, Fig. 7). Gene silencing as well the administration of a commercially available non-selective FGFR inhibitor (Fig. 8, Fig. 9), PD173074 blocks AFP production (Fig. 10). Furthermore, this inhibitor exhibited exquisite anti-proliferative effect on HCC cell lines vs. non-cancerous cell line, HEK293 (data not shown, LD50>50 micromolar, see also Figure 11). Hence, it is postulated that FGFR4 activity contributes to normal-to-tumor progression of HCC and may be a viable target for pharmacological intervention.

### FGFR4 Y367C

The somatic mutation FGFR4 Y367C identified within the extracellular domain FGFR4 Y367C possibly augment receptor activation by receptor dimerization. The novel FGFR4 Y367C mutation was detected as a homozygous genotype in the breast cancer cell line MDA-MB-453, and the affected Y367 residue in the extracellular juxtamembrane domain is highly conserved throughout the FGFR family. Remarkably, homologous substitutions in FGFR1 (Y372C), FGFR2 (Y375C) and FGFR3 (Y373C) were shown to cause various osteogenic deficiency syndromes (Wilkie, A.O., Cytokine Growth Factor Rev 16:187-203 (2005)) through the formation of intermolecular disulfide bonds that force receptor dimerization and activation. Ligand-independent, constitutive receptor activation has been confirmed in vitro for FGFR1 Y372C (White, K.E., et al., Am J Hum Genet 76:361-367 (2005)) and FGFR3 Y373C (d'Avis, P.Y., et al., Cell Growth Differ; 9:71-78 (1998)). Furthermore, the oncogenic potential of the FGFR3 Y373C variant has been demonstrated and was suggested to contribute to tumor progression of multiple myeloma (Chesi, M., Blood, 97:729-736 (2001)). Thus, it is most likely that Y367C as the homologous FGFR4 variant also results in basal receptor activation, which strongly indicates an important role of this mutant in cancer.

### EXAMPLES

### Samples

Samples of primary invasive breast carcinomas were obtained from the archives of the Department of Pathology of the Technical University of Munich, Germany (Prof. H. Hoefler) and the Department of Oncology of the University of Chieti, Italy (Dr. S. Iacobelli). Kidney tissue materials of tumors and healthy tissue as well as prostate cancer tissue were obtained from the Urology Department of the Klinikum Darmstadt, Germany (Prof. S. Peter). 14 cDNAs of normal tissue (spleen, testes, ovary, kidney, skeletal muscle, colon, prostate, bladder, cervix, pancreas, liver, brain, lung, gastric) derived from different individuals were purchased from Ambion.

Genomic DNA of 90 blood samples derived from non-cancer patients was purchased from Coriell Institute for Medical Research (Camden, NJ, USA).

### cDNA Synthesis

Total RNA was isolated according to the method described by Puissant and Houdebine.

Cancer cell lines were cultured according to conditions by the American Type Culture Collection (ATTC, http://www.atcc.org).

After homogenization of the cultured human cancer cells (80 % confluency) or the primary tissue in a denaturing solution (4M guanidine thiocyanate, 25mM sodium citrate, 0.5% Sarkosyl, 0.1M ß-mercaptoethanol, 10mM EDTA), the homogenate was sequentially mixed with 2M sodium acetate (pH 4.0), saturated phenol and finally with chloroform. The mixture was centrifuged and the upper phase was isopropanol precipitated, resuspended in denaturing solution and again reprecipitated with isopropanol. Following ethanol washing the pellet was resuspended in H₂O and incubated at 65°C for 5 min. The quality of the total RNA was tested by gel electrophoresis.

For the extraction of poly(A)⁺RNA, total RNA was denatured at 70 °C (5min) and applied to a oligo-dT-cellulose column along with a washing buffer (10 mM Tris/HCl pH7.4, 0.5M NaCl, 1mM EDTA, 0.5 % SDS). Following several washing steps the poly(A)⁺RNA was eluted (10 mM Tris/HCl pH7.4, 1mM EDTA, 0.5% SDS) and precipitated with ethanol.

The conversion of the poly(A)⁺RNA into the complementary DNA (cDNA) was performed using the AMV reverse transcriptase (Promega AMV-RT) and oligo(dT) polymers and oligonucleotides (dNTP). After the synthesis the cDNA was purified using Qiagen PCR purification columns and eluted in 50µl.

### PCR and Sequencing

For each cell line and control sample, whole cell cDNA was prepared and used for the amplification and direct sequencing of the complete PTK coding region. Primers for PCR amplification (and sequencing) were designed using Primer3 program (http://www-genome.wi.mit.edu/cgi-bin/primer/primer3_www.cgi), and were synthesized by Proligo (Singapore). PCR amplification was performed on cDNA from 265 early passage cell lines, placenta and 14 normal tissues (Ambion). PCR optimization was done in the first step with a cDNA pool of different cell lines, second step with 6/8 different individual cell lines, before being dispensed into a 96-well culture plate. Direct sequencing was done using a 96 capillary automated sequencing apparatus (ABI 3730XL).

### Analysis of Mutations

Sequence traces were assembled and analyzed to identify potential genomic alterations using the Mutation Surveyor software package (SoftGenetics, State College, PA). The tyrosine kinase gene sequences were aligned with the NCBI reference sequence (Fig. 12) and identified alterations were compared with the literature or public databases such as the NCBI SNP database (http://www.ncbi.nlm.nih.gov), the Ensemble Genome Browser (http://www.ensembl.org), the UniProtKB/Swiss-Prot database (http://ca.expasy.org) the SNP500Cancer database, and KinMutBase (http://bioinf.uta.fi/KinMutBase/main_frame.html).

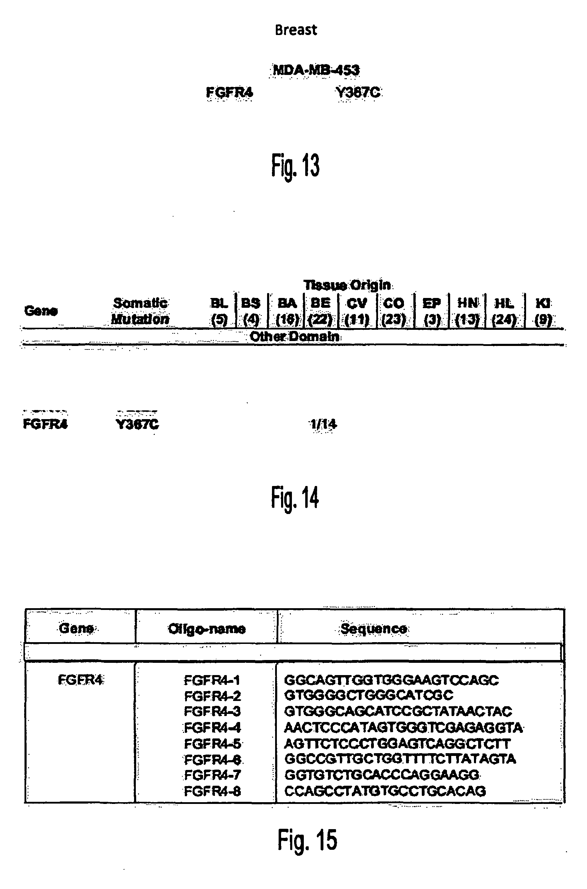

The characterization of cell line can be found in Fig. 13.

### Gene Identification

The coding sequences of the analyzed RTK and TK genes were retrieved from NCBI (www.ncbi.gov). NCBI accession number of FGFR4 is provided in Gbk-file: NM_002011.2.

With intermediate mutation rates for the other tumor cell lines, an accumulation of somatic mutations in PTK transcripts of various cancer cell lines, which may contribute to the progression characteristics of certain cancers, were shown.

As alternative to the allocation to cell line-specific variant profiles, these sequence differences were grouped by genes and PTK subfamilies. Each variation was thereby specified regarding the spectrum of affected cell lines as well as the zygosity status and the presumable somatic versus germline origin, for example,for FGFR4 (Fig. 1).

### Identification of 256 somatic mutations in cancer cell lines

Of all sequence differences, 234 were undetectable in any of the control samples or public databases and were thus defined as somatic mutations. However, because of the lack of cell line-specific normal tissue controls, the possibility cannot be excluded that some actually represent rare germline polymorphisms. The somatic mutations are composed of 210 missense and 2 nonsense single nucleotide substitutions as well as 19 deletions and 3 insertions. While the majority (186) occurred once, 53 were found 2 to 5 times, and 3 in 6 to 10 tumor cell lines. Among the twice occurring somatic mutations, 20 were detected in cell lines originating from the same tumor donor (Fig. 12). They may be considered single mutations, thus adding up to a total of 206 non-recurring mutational events.

The domain localization and tissue distribution of identified somatic mutations are summarized in **Fig. 14****.** Somatic mutations are characterized with regard to their localization within the protein and the tissue origin (BL: bladder; BS: bone and soft tissue; BA: brain; BE: breast; CV: cervix and vulva; CO: colon; EP: endometrium and placenta; HN: head and neck; HL: hematopoietic and lymphoid system; KI: kidney; LI: liver; LU: lung; OV: ovary; PA: pancreas; PR: prostate; SK: skin; ST: stomach; TE: testes; TY: thyroid, NO: normal control samples) of affected cell lines. The first of the paired numerals provided for a given somatic mutation and particular tumor type indicates the number of mutated cell lines, the second numeral refers to the number of expression-positive cell lines. Somatic mutations affecting the activation loop (a), the catalytic loop (c), the P-loop (p) or the pseudokinase (ps) domain are indicated.

### Somatic mutations with possible oncogenic potential

The somatic mutation that the present inventors identified within the extracellular domain of FGFR family member, FGFR4 Y367C (Figs. 2 and 3), possibly augment receptor activation by influencing ligand binding and receptor dimerization, respectively.

The novel FGFR4 Y367C mutation was detected as a homozygous genotype in the breast cancer cell line MDA-MB-453, and the affected Y367 residue in the extracellular juxtamembrane domain is highly conserved throughout the FGFR family. Remarkably, homologous substitutions in FGFR1 (Y372C), FGFR2 (Y375C) and FGFR3 (Y373C) were shown to cause various osteogenic deficiency syndromes (Wilkie, Cytokine Growth Factor Rev 16:187-203 (2005)) through the formation of intermolecular disulfide bonds that force receptor dimerization and activation. Ligand-independent, constitutive receptor activation has been confirmed in vitro for FGFR1 Y372C (White et al., Am J Hum Genet 76:361-367 (2005)) and FGFR3 Y373C (d'Avis et al., Cell Growth Differ 9:71-78 (1998)). Furthermore, the oncogenic potential of the FGFR3 Y373C variant has been demonstrated and was suggested to contribute to tumor progression of multiple myeloma (Chesi et al.; Blood 97:729-736 (2001)). Thus, it is most likely that Y367C as the homologous FGFR4 variant also results in basal receptor activation, which strongly indicates an important role of this mutant in cancer.

### Cell culture, Plasmids

HEK293, Jurkat E6.1, HuH7, HepG2, MCF-7 and MDA-MB-231 cells were purchased from ATCC (Manassas, VA). HEK 293, HuH7 and MCF-7 were maintained in DMEM (high glucose) medium supplemented with sodium pyruvate and 10 % FCS. Jurkat E61 and MDA-MB-231 were maintained in RPMI supplemented with L-glutamine, sodium pyruvate and 10 % FCS. HepG2 was maintained in MEM supplemented with non-essential amino acids, L-glutamine, sodium pyruvate and 10 % FCS. All cell culture reagents were from Invitrogen (Carlsbad, CA) unless otherwise stated.

### Sample preparation

Fifty seven adjacent normal tissue were obtained from resected livers of patients from the National University Hospital (patient's consent for collection of tissue was obtained prior operation). The tumor and normal liver tissues were visually separated. Both tumor and normal tissue were cut into small pieces and flash frozen in liquid nitrogen immediately after being harvested from patients. The frozen tissues were later stored in -80 °C. RNA extraction from frozen tissue was carried out by TriZol method as described previously (Chomczynski, P., & Sacchi, N., Nat Protoc 1:581-5 [2006]).

### mRNA purification

mRNA was purified from total RNA (50 µg per sample) using the Oligotex mRNA kit (Qiagen, Valencia, CA) performed according to manufacturer's protocol. The resulting mRNA from the Oligotex columns was eluted with two volumes of 50 µL of the elution buffer supplied in the kit. To purify and concentrate the eluant, 2 µL of pellet paint (Merck, Darmstadt, Germany) and 10 µL of 3M sodium acetate were first added to enhance visualization of the produce before precipitating overnight with 200 µL of absolute ethanol at -20 °C. The resultant mRNA was pelleted by spinning at 13,000 rpm for 30 min and subsequently washed with another volume of 80 % ethanol. The final precipitate was air-dried and re-dissolved in 10 µL of RNAse-free water.

### First strand cDNA synthesis

The purified mRNA (4 µL) from above was mixed gently with 1 µL of OligoDT15 primer (100 µM, Roche, Basel, Switzerland) in a 1.5 mL microcentrifuge tube and incubated at 70 °C for 2 min. After cooling on ice, 15 µL of reverse transcription mix containing 4 µL of 5X RT buffer, 2.4 µL of MgCl₂, 1 µL dNTP (10 mM), 1 µL RNase inhibitor, 1 µL ImProm-II RT (Promega) and 5.6 µL of water was added. The reaction was maintained at 42 °C for 1 h and was quenched with 75 µL of TE buffer. The resultant cDNAs were purified with QiaQuick PCR purification kit (Qiagen).

### Sequencing and mutational analysis:

Primers for PCR amplification of cDNA samples (and sequencing) were designed using Primer3 program (http://www-genome.wi.mit.edu/cgi-bin/primer/primer3_www.cgi), and were synthesized by Proligo (SigmaAldrich, Singapore). PCR reactions were optimized as previously described for FGFR4. Direct sequencing was done using a 96 capillary automated sequencing apparatus (ABI 3730XL). Sequence traces were assembled and analyzed to identify potential genomic alterations using the Mutation Surveyor software package (SoftGenetics, State College, PA). The entire coding sequence of FGFR4 was aligned to the NCBI reference sequence (NM_002011.2) and identified alterations were compared to known mutations in the literature (in publications) or public databases such as NCBI SNP database (http://www.ncbi.nlm.nih.gov), the Ensemble Genome Browser (http://www.ensembl.org), the UniProtKB/Swiss-Prot database (http://ca.expasy.org) and KinMutBase (http://bioinf.uta.fi/ KinMutBase/main_frame. html).

### Quantitative Real-time PCR

Quantitative RT-PCR was carried out using an Applied Biosystems 7300 Real-time PCR system (ABI, Foster City, CA) with pre-optimized TaqMan Gene Expression Assay for human FGFR4 and human GAPDH as the housekeeping control. The thermal cycling condition included an initial denaturation step at 95 °C for 10 min, followed by 40 cycles at 95 °C for 15 s and 60 °C for 60 s. The samples were prepared in triplicate with 4 µL of prediluted cDNA (2-10 fold) samples each. Data were obtained as an average CT value, and subsequently normalized against GAPDH endogenous control as ΔC_{T}. Expression changes in FGFR4 transcripts between the normal and the corresponding tumor tissue were expressed as fold change using 2^(difference in ΔC_{T} between pairs).

### Ligand stimulation assay

HepG2 or HuH7 cells were seeded into 96-well plates and allowed to attach overnight. They were serum-starved for 24 h before addition of heparin. Two hours later, FGF19 (R&D Systems, Minneapolis, MN, 50-100 ng/mL final concentration) was administered. After 8 hours, aliquots of the supernatant were harvested for AFP ELISA assay as described below. Similar FGF19 stimulations were performed in 10 cm dishes for immunoblot detection of phospho-FRS2α.

### Immunoprecipitation, Immunoblot assay

All fractions collected were assayed for protein concentration using a BCA protein assay kit (Pierce, Rockford, IL). Lysates were pre-cleared by centrifugation at 13 000 r.p.m. for 10 min at 4 °C. For immunoprecipitation, supernatants were diluted with an equal volume of HNTG buffer (Seedorf, K., et al., J Biol Chem. Jun 10; 269(23):16009-16014 (1994)) and subsequently immunoprecipitated using the respective antibodies and 20 µl of protein A/G-Sepharose for 4 h at 4 °C. Precipitates were washed three times with 0.5 ml of HNTG buffer, suspended in SDS sample buffer and boiled for 3 min.

For the immunoblot assay, sample proteins (30-50 µg) or the immunoprecipitated samples were resolved by denaturing electrophoresis using 7.5 % SDS-PAGE and transferred to nitrocellulose membranes for 2 h at 5 V using Trans-Blot SD Semi-Dry Transfer Cell (Bio-Rad). Immunodetection was by chemiluminescence (SuperSignal West Dura Extended, Pierce) using specific antibodies diluted in PBS with 0.05% (v/v) Tween 20 and 5% (w/v) powdered milk. Anti-phospho-FRS2a anti-HA, anti-phospho-MAPK, anti-MAPK, anti-phospho-Stat3, anti-Stat3 were from Cell Signaling Technology (Beverly, MA); anti-Ack1, anti-myc and anti-FGFR4 were from Santa Cruz Biotechnology (Santa Cruz, CA); anti-β-actin controls and anti-TEC were from Abcam (Cambridge, UK); anti-4G10 and anti-TYK2 were from Upstate (Lake Placid, New York, USA); anti-Hsp60, anti-Flag were from Sigma (St Louis, USA). Secondary anti-mouse and anti-rabbit horseradish peroxidase conjugated secondary antibodies (Pierce) were used at 1:10000 dilution.

### AFP ELISA assay

Supernatants from the respective FGF19 stimulation and siRNA experiments were subjected to AFP ELISA assay using the DELFIA hAFP kit (Perkin Elmer, Boston, MA), performed according to manufacturer's protocol. The readout was converted to concentration (ng/mL) using the standard curve derived from the solutions provided in the kit. AFP production by the cells was subsequently normalized to the number of cells in each well. The cell number was determined by ATP bioluminescence Cell-Titer Glo assay (Promega, Madison, WI) using protocol as described by the manufacturer.

### Gene silencing by siRNA

HuH7 cells were grown on 24-well plate to 50% confluence before transfection with siRNA (small interfering RNAs). Custom-made ON-TARGETplus siRNA designed for silencing *FGFR4* (Accession number: NM_002011) expression was purchased from Dharmacon. A microcentrifuge tube containing 1.3 µL of 20 µM siRNA and 40.2 µL complete growth medium was prepared (Tube A). Simultaneously, another tube containing 1 µL oligofectamine (Invitrogen) and 7.5 µL growth medium was also prepared (Tube B). Both tubes were incubated at room temperature for 5 min before combining the contents and left to stand for another 20 min. Next, each well of HuH7 cells was replaced with fresh serum-free medium (200 µL). The combined volume of the siRNA transfection mix (50 µL) was added to the well and incubated at 37 °C. After 4 h, the samples were loaded with another 250 µL of growth medium containing 20 % serum. Immunoblot and AFP ELISA assays were subsequently performed after 72 h incubation of the cells with the FGFR4 silencing complex.

### c-fos gene reporter assay

HEK-293 cells (2 x 10⁴/96-well) were transiently transfected with 0.2 µg of the pfos/luc reporter plasmid (Yamashita et al., Blood 91, 1496-1507 (1998)) and 0.1 µg of expression plasmids for TEC or its mutants. 24 hours after transfection luciferase activity was measured with the use of the dual luciferase assay system (Promega, Madison, WI).

### Ubiquitination Assay

3.5 x 10⁶ Hek 293 cells were seeded on 10 cm dish. Cells were co-transfected with 7.5 µg Myc-tagged Ubiquitin and 10 µg Flag-tagged Ack1, Ack1 S985N, Mop1 and EphA5, using LipofectamineTM 2000 (Invitrogen, San Diego, CA) according to the manufacturer's instructions.

20 hours post transfection, cells are incubated with 10 µM MG132 (Sigma, St Louis, MO) for 8 hours and lysed by RIPA buffer. 500 µg of protein lysate were used for immunoprecipitation with 2 µg anti-myc antibody at 4 degrees Celsius overnight. IP samples were washed and denatured with SDS-lysis buffer (50 mM Tris-HCl pH 6.8, 100 mM DTT, 2 % SDS) and separated on a 7.5 % SDS-PAGE gel. Co-IP proteins were detected using anti-Flag antibodies.

### MG132 Treatment

1 x 10⁶ HepG2 cells were seeded on 6 well plate the day before treatment. Cells were incubated with 10 µM MG132 (Sigma, St Louis, MO) and harvested according to time course. 30 µg cell lysate was separated with a 7.5 % SDS-PAGE gel.

The listing or discussion of a previously published document in this specification should not necessarily be taken as an acknowledgement that the document is part of the state of the art or is common general knowledge.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

One skilled in the art would readily appreciate that the present invention is well adapted to carry out the objects and obtain the ends and advantages mentioned, as well as those inherent therein. Further, it will be readily apparent to one skilled in the art that varying substitutions and modifications may be made to the invention disclosed herein without departing from the scope of the invention. The molecular complexes and the methods, procedures, treatments, molecules, specific compounds described herein are presently representative of preferred embodiments are exemplary and are not intended as limitations on the scope of the invention. Changes therein and other uses will occur to those skilled in the art which are encompassed within the scope of the invention are defined by the claims.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" etc. shall be read expansively and without limitation, and are not limited to only the listed components they directly reference, but include also other non-specified components or elements. As such they may be exchanged with each other. Additionally, the terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. An isolated, enriched, or purified nucleic acid molecule encoding a mutant of FGFR4, wherein the mutant of FGFR4 encoded by the nucleic acid molecule comprises the mutation FGFR4 Y367C (SEQ ID No: 133.

2. A method of identifying a cell having a predisposition to transform into a cancer cell, the method comprising:
- identifying the amino acid at position 367 of the expressed protein kinase FGFR4, wherein the presence of Cysteine at position 367 of the expressed protein kinase FGFR4 instead of Tyrosine indicates an increased predisposition to transform into a cancer cell.

3. The nucleic acid molecule according to claim 1, wherein the nucleic acid molecule is isolated from a natural source, wherein the natural source is preferably mammal, and wherein the mammal is preferably a human.

4. The nucleic acid molecule according to claim 1 br3, wherein the nucleic acid molecule is of recombinant origin or wherein the nucleic acid molecule is RNA or DNA.

5. A nucleic acid probe for the detection of a nucleic acid molecule encoding a mutant kinase polypeptide in a sample, wherein the mutant kinase polypeptide is FGFR4, and wherein said mutant kinase polypeptide encoded by said nucleic acid molecule comprises the mutation FGFR4 Y367C (SEQ ID No: 402), wherein said nucleic acid probe contains a nucleotide base sequence that specifically hybridizes only to a nucleic acid molecule encoding FGFR4 having the Y367C mutation.

6. A method for detecting the presence or amount of nucleic acid molecule encoding a mutant of FGFR4 in a sample, wherein the mutant of FGFR4 encoded by the nucleic acid molecule comprises the mutation FGFR4 Y367C (SEQ ID NO: 133), the method comprising the steps of a) contacting the sample with a nucleic acid probe according to claim 5 under conditions such that hybridization occurs and b) detecting the presence or amount of the probe bound to nucleic acid molecules encoding the mutant of FGFR4.

7. A kit for performing the method of claim 6, including a container means having disposed therein one or more nucleic acid probes according to claim 5.

8. A recombinant cell or tissue comprising a nucleic acid molecule according to any of claims 1, 3 and 4, wherein the nucleic acid molecule is under the control of genomic regulatory elements or heterologous regulatory elements including a heterologous promoter.

9. An isolated, enriched, or purified mutant kinase polypeptide, wherein said mutant kinase polypeptide is FGFR4 and wherein said mutant FGFR4 comprises the mutation FGFR4 Y367C (SEQ ID No: 133), or a fragment thereof.

10. The kinase polypeptide of claim 9, wherein the kinase polypeptide is isolated from a natural source or being of recombinant origin; wherein the natural source is preferably a mammal; and wherein the mammal is preferably a human.

11. A method for detecting the presence and/or amount of at least one mutant of FGFR4 comprising the mutation FGFR4 Y367C (SEQ ID No: 133) in a sample, the method comprising the steps of a) probing the sample with a monoclonal or polyclonal antibody or antibody fragment having specific binding affinity only for a mutant kinase polypeptide according to claim 9, under conditions suitable for kinase-antibody immunocomplex formation and b) detecting the presence and/or amount of the antibody bound to the kinase polypeptide.

12. A kit for performing the method of claim 11, including the antibody or the antibody fragment.

13. A method for identifying a compound that modulates kinase activity in vitro comprising the steps of: (a) contacting a kinase polypeptide according to claim 9 with a test substance; (b) measuring the activity of said kinase polypeptide; and (c) determining whether said substance modulates the activity of said kinase polypeptide.

14. A method for the diagnosis of a proliferative disease or disorder or the risk prediction of developing a proliferative disease or disorder in a subject, said disease or disorder being **characterized by** an abnormality in a signal transduction pathway due to aberrant protein kinase function, wherein said method comprises: (a) contacting a biological sample obtained from the subject with a nucleic acid probe which hybridizes under hybridization assay conditions to a target region of a nucleic acid molecule encoding a mutant kinase polypeptide according to claim 9; and (b) detecting the presence or amount of the probe:target region hybrid as an indication of or predisposition to the disease or disorder.

## Patentansprüche

1. Ein isoliertes, angereichertes oder aufgereinigtes Nukleinsäuremolekül, das eine Mutante von FGFR4 kodiert, wobei die Mutante von FGFR4, die durch das Nukleinsäuremolekül kodiert wird, die Mutation FGFR4 Y367C (SEQ ID NO: 133) umfasst.

2. Ein Verfahren zum Identifizieren einer Zelle, die eine Prädisposition für eine Transformation in eine Krebszelle besitzt, das Verfahren umfassend:
- Identifizieren der Aminosäure an Position 367 der exprimierten Proteinkinase FGFR4, wobei die Anwesenheit von Cystein an Position 367 der exprimierten Proteinkinase FGFR4 anstelle von Tyrosin eine erhöhte Prädisposition für eine Transformation in eine Krebszelle anzeigt.

3. Das Nukleinsäuremolekül nach Anspruch 1, wobei das Nukleinsäuremolekül aus einer natürlich vorkommenden Quelle isoliert wird, wobei die natürlich vorkommende Quelle vorzugsweise ein Säugetier ist, wobei das Säugetier vorzugsweise ein Mensch ist.

4. Das Nukleinsäuremolekül nach Anspruch 1 oder 3, wobei das Nukleinsäuremolekül rekombinanter Herkunft ist oder wobei das Nukleinsäuremolekül RNA oder DNA ist.

5. Eine Nukleinsäuresonde zum Nachweis eines Nukleinsäuremoleküls, das ein mutiertes Kinasepolypeptid kodiert, in einer Probe, wobei das mutierte Kinasepolypeptid FGFR4 ist und wobei das mutierte Kinasepolypeptid, das durch das Nukleinsäuremolekül kodiert wird, die Mutation FGFR4 Y367C (SEQ ID NO: 402) umfasst, wobei die Nukleinsäuresonde eine Nukleotidsequenz beinhaltet, die spezifisch nur an ein Nukleinsäuremolekül hybridisiert, das FGFR4 kodiert und die Y367C Mutation besitzt.

6. Ein Verfahren zum Nachweis der Anwesenheit oder Menge eines Nukleinsäuremoleküls, das eine Mutante von FGFR4 kodiert, in einer Probe, wobei die Mutante von FGFR4, die durch das Nukleinsäuremolekül kodiert wird, die Mutation FGFR4 Y367C (SEQ ID NO: 133) umfasst, das Verfahren umfassend die Schritte a) das in Kontakt bringen der Probe und der Nukleinsäuresonde nach Anspruch 5 unter Bedingungen, so dass eine Hybridisierung erfolgt, und b) Nachweis der Anwesenheit oder Menge der Sonde, die an das Nukleinsäuremolekül, das die Mutante von FGFR4 kodiert, gebunden ist.

7. Ein Kit zum Durchführen des Verfahrens nach Anspruch 6, das einen Behälter einschließt, in dem ein oder mehrere Nukleinsäuresonden nach Anspruch 5 vorhanden sind.

8. Eine rekombinante Zelle oder ein rekombinantes Gewebe umfassend ein Nukleinsäuremolekül nach einem der Ansprüche 1, 3 und 4, wobei das Nukleinsäuremolekül unter der Kontrolle eines genomischen Regulatorelements oder eines heterologen Regulatorelements, einschließlich eines heterologen Promoters, steht.

9. Ein isoliertes, angereichertes oder aufgereinigtes mutiertes Kinasepolypeptid, wobei das mutierte Kinasepolypeptid FGFR4 ist und wobei das mutierte FGFR4 die Mutation FGFR4 Y367C (SEQ ID NO: 133) umfasst oder ein Fragment davon.

10. Das Kinasepolypeptid nach Anspruch 9, wobei das Kinasepolypeptid aus einer natürlich vorkommenden Quelle isoliert wird oder rekombinanten Ursprung ist; wobei die natürlich vorkommende Quelle vorzugsweise ein Säugetier ist, wobei das Säugetier vorzugsweise ein Mensch ist.

11. Ein Verfahren zum Nachweis der Anwesenheit und/oder Menge mindestens einer Mutante von FGFR4, umfassend die Mutation Y367C (SEQ ID NO: 133) in einer Probe, wobei das Verfahren die Schritte umfasst a) Untersuchen der Probe mit einem monklonalen oder polyklonalen Antikörper oder einem Antikörperfragment, die eine spezifische Bindungsaffinität ausschließlich für ein mutiertes Kinasepolypeptid nach Anspruch 9 besitzen unter Bedingungen, die für die Bildung eines Kinase-Antikörper-Immunkomplexes geeignet sind und b) Nachweis der Anwesenheit und/oder Menge des Antikörpers, der an das Kinasepolypeptid gebunden ist.

12. Ein Kit zum Durchführen des Verfahrens nach Anspruch 11, das den Antikörper oder das Antikörperfragment einschließt.

13. Ein Verfahren zum Identifizieren einer Verbindung, die in vitro die Kinaseaktivität modulieren kann, wobei das Verfahren die Schritte umfasst: (a) in Kontakt bringen eines Kinasepolypeptids nach Anspruch 9 und einer zu testenden Substanz; (b) Messen der Aktivität des Kinasepolypeptids; und (c) Bestimmen, ob die Substanz die Aktivität des Kinasepolypeptids moduliert.

14. Ein Verfahren zur Diagnose einer proliferativen Erkrankung oder Störung oder zur Vorhersage des Risikos eine proliferative Krankheit oder Störung zu entwickeln in einem Subjekt, wobei die Krankheit oder Störung sich aufgrund einer abweichenden Proteinkinasefunktion durch eine Abnormalität in einem Transduktionssignalweg auszeichnet, wobei das Verfahren umfasst: (a) in Kontakt bringen einer biologischen Probe, die von dem Subjekt erhalten wurde, mit einer Nukleinsäuresonde, die unter Hybridisierungsassay-Bedingungen an eine Zielregion eines Nukleinsäuremoleküles, das ein mutiertes Kinasepolypeptid nach Anspruch 9 kodiert, hybridisiert; und (b) Nachweis der Anwesenheit oder Menge eines Sonden:Zielregion Hybrids als Hinweis auf die Krankheit oder Störung oder als Hinweis auf eine Prädisposition für diese Krankheit oder Störung.

## Revendications

1. Molécule d'acide nucléique enrichie ou purifiée isolée, codant un mutant de FGFR4, le mutant de FGFR4 codé par la molécule d'acide nucléique comprenant la mutation FGFR4 Y367C (SEQ ID N° : 133).

2. Procédé destiné à identifier une cellule qui présente une prédisposition à se transformer en une cellule cancéreuse, le procédé comprenant :
- l'identification de l'acide aminé à la position 367 de la protéine kinase exprimée FGFR4, dans lequel la présence de cystéine à la position 367 de la protéine kinase exprimée FGFR4 au lieu de tyrosine indique une prédisposition accrue à une transformation en une cellule cancéreuse.

3. Molécule d'acide nucléique selon la revendication 1, laquelle molécule d'acide nucléique est isolée à partir d'une source naturelle, la source naturelle étant de préférence un mammifère, et le mammifère étant de préférence un être humain.

4. Molécule d'acide nucléique selon la revendication 1 ou 3, laquelle molécule d'acide nucléique est d'origine recombinante ou laquelle molécule d'acide nucléique est l'ARN ou l'ADN.

5. Sonde d'acide nucléique destinée à la détection d'une molécule d'acide nucléique codant un polypeptide de kinase mutant dans un échantillon, où le polypeptide de kinase mutant est FGFR4, et où ledit polypeptide de kinase mutant codé par ladite molécule d'acide nucléique comprend la mutation FGFR4 Y367C (SEQ ID N° 402), ladite sonde d'acide nucléique contenant une séquence de bases nucléotidiques qui s'hybride de manière spécifique seulement à une molécule d'acide nucléique qui code FGFR4 présentant la mutation Y367C.

6. Procédé destiné à détecter la présence ou la quantité de molécule d'acide nucléique codant un mutant de FGFR4 dans un échantillon, où le mutant de FGFR4 codé par la molécule d'acide nucléique comprend la mutation FGFR4 Y367C (SEQ ID : N° 133), le procédé comprenant les étapes consistant à : a) mettre en contact l'échantillon avec une sonde d'acide nucléique selon la revendication 5 dans des conditions telles qu'une hybridation se produise ; et b) détecter la présence ou la quantité de sonde liée aux molécules d'acide nucléique codant le mutant de FGFR4.

7. Kit destiné à exécuter le procédé selon la revendication 6, comprenant des moyens formant contenant dans lesquels sont disposées une ou plusieurs sondes d'acide nucléique selon la revendication 5.

8. Cellule ou tissu recombinants comprenant une molécule d'acide nucléique selon l'une quelconque des revendications 1, 3 et 4, laquelle molécule d'acide nucléique est sous le contrôle d'éléments de régulation génomiques ou d'éléments de régulation hétérologues comprenant un promoteur hétérologue.

9. Polypeptide de kinase mutant enrichi ou purifié isolé, où ledit polypeptide de kinase mutant est FGFR4 et où ledit mutant FGFR4 comprend la mutation FGFR4 Y367C (SEQ ID N° 133), ou un fragment de celle-ci.

10. Polypeptide de kinase selon la revendication 9, lequel polypeptide de kinase est isolé à partir d'une source naturelle ou est d'origine recombinante, la source naturelle étant de préférence un mammifère, et le mammifère étant de préférence un être humain.

11. Procédé destiné à détecter la présence et/ou la quantité d'un mutant de FGFR4 au moins qui comprend la mutation FGFR4Y367C (SEQ ID N° 133) dans un échantillon, le procédé comprenant les étapes consistant à : a) sonder l'échantillon avec un anticorps ou un fragment d'anticorps monoclonal ou polyclonal qui présente une affinité de liaison spécifique seulement pour un polypeptide de kinase mutant selon la revendication 9, dans des conditions appropriées à la formation d'immunocomplexes kinase-anticorps ; et b) détecter la présence et/ou la quantité de l'anticorps lié au polypeptide de kinase.

12. Kit destiné à exécuter le procédé selon la revendication 11, comprenant l'anticorps ou le fragment d'anticorps.

13. Procédé destiné à identifier un composé qui module une activité de kinase in vitro comprenant les étapes consistant à : (a) mettre en contact un polypeptide de kinase selon la revendication 9 avec une substance d'essai ; (b) mesurer l'activité dudit polypeptide de kinase ; et (c) déterminer si ladite substance module l'activité dudit polypeptide de kinase.

14. Procédé destiné au diagnostic d'une maladie ou d'un trouble capable de proliférer ou à une prévision de risque de développer une maladie ou un trouble capable de proliférer chez un sujet, ladite maladie ou ledit trouble étant **caractérisé par** une anomalie dans une voie de transduction du signal due à la fonction de protéine kinase anormale, ledit procédé comprenant les étapes consistant à : (a) mettre en contact un échantillon biologique obtenu à partir du sujet avec une sonde d'acide nucléique qui s'hybride dans des conditions de dosage d'hybridation à une région cible d'une molécule d'acide nucléique codant un polypeptide de kinase mutant selon la revendication 9 ; et (b) détecter la présence ou la quantité d'hybride dans la région sonde : cible en tant qu'indication de la maladie ou du trouble ou en tant que prédisposition à la maladie ou au trouble.
